(19) Europäisches Patentamt
European Patent Office
Office européen des brevets

(11) EP 3 502 242 A1

(12) EUROPEAN PATENT APPLICATION

(43) Date of publication:
**26.06.2019 Bulletin 2019/26**

(21) Application number: **17208848.6**

(22) Date of filing: **20.12.2017**

(51) Int Cl.:
***C12N 9/24*** *(2006.01)* ***D21C 5/00*** *(2006.01)*
***D21H 17/00*** *(2006.01)* ***A23K 10/14*** *(2016.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Technische Universität München**
**80333 München (DE)**

(72) Inventors:
- **Graubner, Sigird**
  **81377 München (DE)**
- **Zverlov, Vladimir**
  **81379 München (DE)**
- **Schwarz, Wolfgang**
  **80637 München (DE)**
- **Hauf, Waldemar**
  **81479 München (DE)**
- **Andreeßen, Björn**
  **85356 Freising (DE)**
- **Schulte, Louis Philipp**
  **80634 München (DE)**
- **Liebl, Wolfgang**
  **85356 Freising (DE)**

(74) Representative: **Maikowski & Ninnemann**
**Patentanwälte Partnerschaft mbB**
**Postfach 15 09 20**
**10671 Berlin (DE)**

Remarks:
Amended claims in accordance with Rule 137(2) EPC.

# (54) NEW XYLANASE WITH IMPROVED THERMOSTABILITY AND INCREASED ENZYME ACTIVITY ON ARABINOXYLAN

(57) The present invention relates to novel polypeptides with xylanase activity, especially xylanase variants, such as genetically engineered xylanase variants, which show improved thermostability, improved resistance against acid treatment and increased enzyme activity on arabinoxylan. The invention includes the use of said polypeptides in applications, such as for food or feed, for brewing or malting, for the treatment of xylan containing raw materials like grain-based materials, e.g. for the production of biofuels or other fermentation products, including biochemicals, and/or for the wheat gluten-starch separation industry, and methods using these polypeptides, as well as compositions (such as feed additive compositions) comprising said polypeptides.

EP 3 502 242 A1

## Description

### Field of the invention

The present invention relates to novel polypeptides with xylanase activity, especially xylanase variants, such as genetically engineered xylanase variants, which show improved thermostability, improved resistance against acid treatment and increased enzyme activity on arabinoxylan. The invention includes the use of said polypeptides in applications, such as food or feed, for brewing or malting, for the treatment of xylan containing raw materials like grain-based materials, e.g. for the production of biofuels or other fermentation products, including biochemicals, and/or for the wheat gluten-starch separation industry, and methods for using these polypeptides, as well as compositions (such as feed additive compositions) comprising said polypeptides.

### Background of the invention

Endo-beta-1,4-xylanase or 4-beta-D-xylan xylanohydrolase (EC 3.2.1.8), referred herein as xylanase, is the designation given to a class of enzymes degrading by endohydrolysis of (1-4)-beta-D-xylosidic linkages the linear polysaccharide beta-1,4-xylan into shorter oligomers. Such enzymes consequently break down hemicellulose, one of the major components of plant cell walls and thus reduce the viscosity of biomass, modifying it's technicophysical properties in biotechnological applications. Xylanases have been used for many years in various industrial applications such as in the production of food or feed, in brewing or malting, in the treatment of arabinoxylan containing raw materials like grain-based materials, e.g. in the production of biofuel or other fermentation products, including biochemicals (e.g. bio-based isoprene), and/or in the wheat gluten starch separation industry, and methods using these xylanases, as well as compositions (such as feed additive compositions) comprising said xylanases.

A common characteristic in all of these applications are the challenging conditions the enzymes have to cope with. For example, high temperatures decrease the effective utility of the presently available xylanases under industrial conditions. In animal feed applications, suitable xylanase enzymes may increase the digestible (=utilizable) part of the biomass in animal feed. Biomass, such as corn, wheat, and DDGS, used for animal feed, comprises two fractions of arabinoxylan, namely the water un-extractable arabinoxylans (WU-AX) and the water extractable arabinoxylans (WE-AX). Useful xylanases must have not only the capability to degrade WU-AX present in the cell walls; they thereby increase the release of encapsulated nutrients. In addition, they also must have the ability to reduce the viscosity caused by the soluble fraction. In addition to high bio-efficacy, useful xylanases also need good product properties such as stability at low pH values and stability against heat processing.

A further desirable property of enzymes used in feed is pepsin resistance. Pepsin is a digestive protease excreted by the animal in the first part of the digestive system. Pepsin degrades proteins. This protease digestion makes proteins available as a source of essential nutrients for the animal. The exogenous enzymes, i.e. enzymes added to the feed, are also proteins and they would be degraded if they are susceptible to degradation by pepsin. This, as it is the case with most enzymes, would destroy the required enzyme activity. Thus, xylanases for use in animal feed applications are only useful it they are resistant to pepsin degradation.

Stability against high temperature is another important feature of a xylanase in order to be useful as a feed additive. It is well known that pelleting increases the digestibility of the starch fraction (Carre et al., 1987). Besides the higher bioavailability of some nutrients there is also less feed waste, a more uniform nutrition and improved feed handling because of reduced dustiness. Pelleting also becomes more important in the context of food safety. Most microorganisms are sensitive to heat under conditions of high moisture. Therefore the feed industry increasingly uses steam pelleting in order to reduce the microbiological load of the feed. For a relatively short time during the feed pelleting process heat (e.g. 30 sec at about 80 °C, WO2008063309) is applied. Appropriate xylanases must tolerate this high temperature without showing denaturation. However, the actual catalytic activity of the enzyme is needed at lower temperatures (e.g. 37 °C). Consequently, the enzyme should not be inactivated irreversibly at high temperatures, while it has to be active at lower temperatures.

Other important industrial applications besides animal nutrition are pulp bleaching, modification of textile fibers (Prade, 1996), and cereal or lignocellulose conversion to solvents and biochemicals such as ethanol. A common characteristic in all these applications are the challenging conditions the enzymes have to cope with. High temperatures, and a pH, which substantially differs from the optimal pH of many xylanases, decrease the effective utility of the presently available xylanases in industrial applications.

In pulp bleaching, the material arising from the alkaline wash has a high temperature (>80 °C) and a high pH (>10). None of the commercially available xylanases is resistant against these conditions. The pulp must be cooled and the alkaline pH be neutralized in order to treat the pulp with presently available xylanases. This results in increased costs. A higher process temperature at higher pH would be helpful to overcome these disadvantages of conventional processes. Solvents, such as ethanol, or biochemicals produced from cereal or lignocellulose starts in most cases with a heating step to decontaminate the substrate and to make the substrate accessible for enzymatic degradation. Xylanases must survive high temperatures in order to reliably reduce the slurry viscosity without causing high cooling costs. Protein engineering has been used - sometimes successively - to stabilize xylanases to resist the denaturing effect of high temperature and unfavorable pH conditions.

Several thermostable, alkaliphilic and acidophilic xylanases have been found and cloned from thermophilic organisms (Bodie et al., 1994; Fukunaga et al., 1998; Dutta et al., 2007; Chi et al., 2012; Prakasch et al. 2012). However, production of economical quantities of these enzymes has in most cases proven to be at least difficult. Therefore the *T. reesei* xylanase II, which is not as such thermostable or alkaliphilic, is in industrial use because it can be produced at low cost in large quantities.

Accordingly, the need exists for novel xylanase enzymes having high bio-efficacy and suitable product properties, including being stable against heat processing. The problem of the invention is therefore the provision of new xylanase enzymes with improved properties for use in industrial processes. This problem is solved by providing novel enzyme variants according to claim 1. The enzyme variants of the present invention have excellent biochemical properties relevant for e.g. feed production cereal and lignocellulose conversion.

## Summary of the invention

The invention provides a polypeptide, preferably a GH11 enzyme, with xylanase activity, in which at least one, preferably two or three carbohydrate binding modules (CBMs) are deleted by genetic engineering, and which shows an improved enzyme profile.

The GH11 xylanase provided by the invention was originally isolated from *Clostridium stercorarium.* It was unexpectedly discovered that deleting the CBMs of the parent enzyme leads to an improved thermostability, improved resistance against acid treatment and increases enzyme activity of the genetically engineered enzyme.

In a preferred embodiment, the GH11 enzyme provided by the invention comprises, essentially consists of or consists of a polypeptide which has at least 75 % amino acid sequence identity to the polypeptide according to SEQ ID NOs: 2 or 3, where two or three CBMs are deleted, respectively. Most preferably the GH11 enzyme provided by the invention comprises, essentially consists of or consists of a polypeptide which has at least 75 % amino acid sequence identity to the polypeptide according to SEQ ID NO: 3.

In a further embodiment, the GH11 enzyme of the invention, in particular the polypeptide according to SEQ ID NO: 3, shows improved characteristics, such as

- resistance against acid treatment; and/or
- resistance against pepsin degradation; and/or
- an about 1.6 fold increased enzyme activity and/or
- an increase of the $Tm_{on}$ from 67 °C to over 80 °C; and/or
- enzyme activity in a broad temperature range from 37 °C to 85 °C; and/or
- Sufficient enzyme activity in a broad pH range from pH 5.0 to 9.5.

In a further embodiment, the invention provides a nucleic acid molecule comprising, consisting essentially of or consisting of a nucleic acid sequence of SEQ ID NO: 9 or 10 encoding the GH11 enzyme according to SEQ ID NO: 2 or 3.

In a further embodiment, the invention provides an expression vector comprising the nucleic acid molecule of SEQ ID NO: 9 or 10 encoding the GH11 enzyme according to SEQ ID NO: 2 or 3.

The invention further provides a host cell comprising the nucleic acid sequence of SEQ ID NO: 9 or 10 or the expression vector comprising said nucleic acid sequence, wherein said host cell expresses the GH11 enzyme according to SEQ ID NO: 2 or 3.

The invention also relates to a method for producing an enzyme of SEQ ID NOs: 2 or 3, preferably of SEQ ID NO: 3, the method comprising culturing a host cell as claimed in claim 8 under conditions permitting the production of the enzyme, and recovering the enzyme from the culture.

In a further embodiment, the invention provides a composition for addition to biomass or hemicellulose containing material, said composition comprising a GH11 enzyme, wherein said GH 11 enzyme comprises, essentially consists of or consists of a polypeptide which has at least 75 % amino acid sequence identity to the polypeptide according to SEQ ID NO 1, 2 or 3, and optionally at least one formulating agent, excipient, stabilizer and/or a preservative.

In a further embodiment of the invention, the GH11 enzyme of the invention or the composition comprising said GH11 enzyme modifies the content of hemicellulose components, in particular the xylan content, to loosen up e.g. compact structure or to reduce high viscosity of biomass or hemicellulose containing material.

The invention relates further to the use of the GH11 enzyme of the invention or the composition comprising said GH11 enzyme in the production of animal feed, pulp and paper, bioenergy and in the brewery.

## Detailed description of the invention

### Definitions

Biomass according to this invention means biomass derived from cell walls unicellular and multicellular organisms from the eukaryotic kingdoms protista (protoctista), fungi and plantae (viridiplantae) comprising vascular plants, mosses, liverworts, hornworts, lichens, ferns, fungi, rhodophyta, glaucophyta, and green algae, whether the material is pretreated or not. From the plants, all parts such as roots, leafs, fruits, stem, are included.

"Pretreatment" according to this invention means treating biomass with mechanical, chemical or physical methods, comprising chopping, crushing, milling, grinding, sonication, heat, steam explosion, solvents, chemicals, acidic or alkaline exposure, liquefaction or drying or a combination of those. Treatment can be performed on disrupted or undisrupted biomass.

Hemicellulose according to this invention is defined as polysaccharides that can be prepared by alkaline extraction of plant tissues. Some of the main polysaccharides that constitute hemicellulose are xylan, glucuronoxylan, arabinoxylan, glucomannan, mixed-linkage beta-glucan and xyloglucan. These soluble, partially soluble or insoluble polysaccharides are essential components of plant cell walls and the major cause for the viscosity of hackled or crushed biomass slurries.

Hemicellulose containing material according to this invention means any substrate comprising hemicellulose in any concentration; this material may be liquefied, partially liquefied or dried and may be in the form of e.g. as slurry, suspension, solution, pulp, paste, batter, dough, mash, process or waste water, powder, granulate, pressed pellets etc.

Conversion of material containing hemicellulose according to this invention is defined as reducing the polymeric degree of hemicellulosic polysaccharides comprising but not limited to xylan, glucuronoxylan, arabinoxylan, beta-glucan (including mixed-linkage beta-glucan) and xyloglucan. These polysaccharides may or may not be derivatised with chemical side groups such as esters (acetyl or feruloyl esters) etc.

"Hemicellulolytic activity" according to the invention is defined as the capability of an enzyme to hydrolyze hemicellulose. Enzymes that depolymerize these polysaccharides by hydrolytic activity are called hemicellulases. Xylanases are one representative class of enzymes belonging to the hemicellulase group.

"Xylanase activity" according to the invention is defined as the capability of an enzyme to degrade the linear polysaccharide beta-1,4-xylan backbone of xylans into shorter oligosaccharides. In particular, according to the invention, xylanase activity means altering the polymeric xylan content in a biomass source to overcome the limitations of material containing hemicellulose such as high viscosity and compact inaccessible structures. Altering the polymeric xylan content means in one embodiment the reduction of the content of polymeric xylan. In another embodiment altering the polymeric xylan content means in the reduction of the content of polymeric arabinoxylan. In another embodiment, altering the polymeric arabinoxylan content means changing the ratio from insoluble (WU-AX) to soluble (WE-AX) arabinoxylan.

"GH11 xylanase" or "GH11 enzyme" according to this invention is defined according to the classification of enzymes into the glycoside hydrolase GH families following the criteria disclosed in the Carbohydrate-Active enZYmes Database (CAZy, http://www.cazy.org/Glycoside-Hydrolases.html): The majority of xylanases can be found in the GH families 5, 7, 8, 10, 11 and 43 (Lombard et al., 2013); however, according to the Carbohydrate-Active enZYmes Database (CAZy) they also appear in other families such as 16, 51, 52, 62 (Adelsberger et al., 2004; Bouraoui et al., 2016; Collins et al., 2005). The most prominent enzyme families for xylanases are GH10 and GH11, which differ significantly in both, their physicochemical properties (including protein structure and folding) and their substrate specificity. GH Family 11 xylanases generally have a low molecular weight and higher pi compared to family 10 xylanases (Kolenová et al. 2006; Collins et al. 2005, Biely et al., 1997). The secondary structure of GH11 enzymes shows a beta-jelly role type of folding. This three-dimensional structure of the enzymes can be predicted from the sequence, and proteins can have an identical fold even if there is very low sequence identity. The active site and the substrate binding amino acid residues are relatively well conserved even though these conserved amino acid residues are rather short sequences in respect to the complete protein sequence.

A CBM module is a non-catalytic carbohydrate-binding module. It is defined as a "contiguous amino acid sequence within a carbohydrate-active enzyme with a discreet fold having carbohydrate-binding activity" (http://www.cazy.org/Carbohydrate-Binding-Modules.html). CBMs were previously known as cellulose-binding domains, until it was found that most of them can also bind other polysaccharides. CBMs are classified into numerous families, based on amino acid sequence similarity. There are currently 64 families of CBMs in the CAZy database which have binding affinity to different soluble or insoluble polysaccharides.

A module is defined as a conserved part of a given protein and has a defined sequence (within similarity boundaries) and (tertiary) structure that can evolve, function, and exist independently of the rest of the protein chain. Each module forms a compact three-dimensional structure and often can be independently stable and folded.

The term "parent enzyme" means a xylanase, preferably a GH11 xylanase, to which an alteration is made to produce a modified enzyme of the present invention. In one embodiment the parent enzyme is a GH11 xylanase. Suitably, the parent enzyme may be a naturally occurring (wild-type) polypeptide or a variant or fragment thereof. In a preferred embodiment the parent enzyme is a naturally occurring (i.e. wildtype) polypeptide.

**Thermostability**

The term "thermostability" is the ability of an enzyme to resist irreversible inactivation (usually by denaturation) at a relatively high temperature at a given pH.

The thermostability of a xylanase (e.g. a modified xylanase) in accordance with the present invention may be determined using the following "assay for measurement of thermostability".

There are many ways of measuring thermostability. It can be measured directly by diluting the enzyme and incubating it with a fluorescent, environment-sensitive dye or directly measuring intrinsic tryptophan fluorescence emission. For this fluorescence emission is monitored while the mixture is gradually heated to 98 °C. Denaturation exposes hydrophobic residues to the solvent where these interact with environment-sensitive dye altering its fluorescence emission spectrum, or denaturation exposes tryptophan residues to the solvent altering its fluorescence emission spectrum. Fluorescence emission intensity is plotted against temperature and fitted using the Boltzmann equation. Parameters derived from the Boltzmann equation can then be used to determine the $Tm_{50}$ which describes the temperature at which 50 % of the enzyme is denatured. Alternatively the values obtained from the Boltzmann equation can be used to calculate the $Tm_{on}$ which describes the temperature at which 1 % of the enzyme is in the denatured state. Both $Tm_{50}$ and $Tm_{on}$ can be used to describe the thermostability of an enzyme as described in example 6.

An indirect way of measuring thermostability may be incubating enzyme samples without substrate for a defined period of time (e.g. 1 to 30 min, such as 5 min) at an elevated temperature compared to the temperature at which the enzyme is stable for a longer time of up to days. Following the incubation at elevated temperature the enzyme sample is assayed for residual activity at the permissive temperature.

By indirectly measuring thermostability enzyme inactivation can be measured as function of temperature. Here enzyme samples are incubated without substrate for a defined period of time (e.g. 1 to 30 min, such as 5 min) at various temperatures and following incubation assayed for residual activity at the permissive temperature, e.g. at a temperature in the range of 37 to 80 °C, such as 37 °C, 50 °C, 60 °C, 70 °C, 80 °C or higher, such as 85 °C or 90 °C or higher. Residual activity at each temperature is calculated as relative to a sample of the enzyme that has not been incubated at the elevated temperature. The resulting thermal denaturation profile (temperature versus residual activity) can be used to calculate the temperature at which 50 % residual activity is obtained. This value is defined as the Tm value.

Even further, by indirect measuring thermostability can be assesed as enzyme inactivation as function of time. Here enzyme samples are incubated without substrate at a defined elevated temperature (e.g. 75 °C) for various time periods (e.g. between 10 sec and 30 min) and following incubation assayed for residual activity at the permissive temperature of e.g. at a temperature in the range of 25 to 80 °C, such as 30 °C, 40 °C, 50 °C, 60 °C, 70 °C or higher, such as at 80 °C or 90 °C or higher. Residual activity at each temperature is calculated as relative to an enzyme sample that has not been incubated at the elevated temperature. The resulting inactivation profile (time versus residual activity) can be used to calculate the time at which 50 % residual activity is obtained. This value is usually given as T1/2.

In one embodiment, an enzyme is considered to be thermostable in accordance with the present invention, if it has a $Tm_{on}$ value of 70 °C or higher, preferably 75 °C or higher, even more preferably 80 °C or higher (at pH 7.0), wherein the $Tm_{on}$ value is the temperature at which 1 % of the enzyme is in a denatured state. This $Tm_{on}$ value may be measured in accordance with the assay for measurement of thermostability as taught herein.

In one embodiment, an enzyme is considered to be thermostable in accordance with the present invention, if it has a $Tm_{50}$ value of 80 °C or higher, preferably 85 °C or higher (at pH 7.0), wherein the $Tm_{50}$ value is the temperature at which 50 % of the enzyme is denatured. This $Tm_{50}$ value may be measured in accordance with the assay for measurement of thermostability as taught herein.

In one embodiment, an enzyme is considered to be thermostable in accordance with the present invention, if it has a Tm value between 80 °C and 90 °C, in a preferred embodiment 85 °C or higher (at pH 6.5), wherein the Tm value is the temperature at which 50 % residual activity is obtained after 5 min incubation. This Tm value may be measured in accordance with the assay for measurement of thermostability as taught herein.

Preferably, the enzyme having xylanase activity, e.g. the GH11 xylanase enzyme (such as the parent or modified GH11 xylanase enzyme) or a fragment thereof according to the present invention (or composition comprising same) can withstand a heat treatment (e.g. during the pelleting process for example) of up to 75 °C. or higher; preferably between 80 °C and 90 °C, more preferably of up to 85 °C (at pH 7.0). The heat treatment may be performed for up to 30 sec; up to 1 minute, up to 5 minutes; up to 10 minutes; up to 30 minutes; up to 60 minutes. To withstand such heat treatment means that at least 50 % of the enzyme that was present/active in the additive before heating to the specified temperature, is still active after cooling to ambient temperature.

These are examples for measuring thermostability. Thermostability can also be measured by other methods. Preferably, thermostability is assessed by use of the "Assay for measurement of thermostability" as taught hereinabove.

In contradistinction to thermostability, thermoactivity is defined as enzyme activity as a function of temperature. To determine thermoactivity, enzyme samples may be incubated (assayed) for the period of time defined by the assay at various temperatures in the presence of a substrate. Enzyme activity is obtained during or immediately after incubation as defined by the assay (e.g. reading an OD-value which reflects the amount of formed reaction product) or as described in example 5. The temperature, at which the highest enzyme activity is obtained, is the temperature optimum of the enzyme at the given assay conditions. The enzyme activity obtained at each temperature can be calculated relative to the enzyme activity obtained at optimum temperature. This will provide a temperature profile for the enzyme at the given assay conditions.

In the present application, thermostability is not the same as thermoactivity.

In a preferred embodiment, the enzyme having xylanase activity, e.g. the GH11 xylanase enzyme, such as the parent or modified GH11 xylanase enzyme of SEQ ID NOs: 2 and 3, preferably the xylanase enzyme of SEQ ID NOs: 3, or a fragment thereof according to the present invention has a Tm value between 80 °C and 90 °C, in a more preferred embodiment between 85 °C and 90°C (at pH 6.5) wherein the Tm value is the temperature at which 50 % residual activity is obtained after 5 min incubation, has a $Tm_{on}$ value of 70 °C, preferably 75 °C, even more preferably 80 °C or higher, most preferably of 81.2 °C (at pH 7.0), wherein the $Tm_{on}$ value is the temperature at which 1 % of the enzyme is in a denatured state, and has a $Tm_{50}$ value of 80 °C, preferably 85 °C or higher, most preferably of 85.7 °C (at pH 7.0), wherein the $Tm_{50}$ value is the temperature at which 50 % of the enzyme is denatured.

## Acidic Treatment

The acidic stability profiles of the polypeptide of SEQ ID NO: 1 and SEQ ID NO: 3 was measured by pre-incubating the enzyme samples in buffer and pH range of 2.5-5.5 such as 5.0,4.5,4.0, 3.5, 3.0 or 2.5 or less for 30,45, 60, 90 and 120 min and subsequently measuring the residual activity by the xylanase activity method as described in Example 5. Enzyme activity measured without pre-incubation was set to 100 % and the residual enzyme activity of each variant at each pH was calculated as relative to this. The GH11 enzyme of the invention is regarded as acidic stable, when an enzyme activity equal to or higher than 70 % is retained after treatment at low pH values. As shown in Figure 5, over 70% of the SEQ ID NO: 3 xylanase activity remained after 2 hours at the low pH.

In a preferred embodiment, the enzyme having xylanase activity, e.g. the genetically engineered GH11 xylanase enzyme or a fragment thereof according to the present invention tolerates acidic treatment in the range of acidic pH 2.5 to 5.5 preferably in the range of 2.5 to 4.0, such as 3.5.

## Formulation and Additives

The GH11 xylanase may be added to the biomass or hemicellulose containing material in a form selected from the group consisting of a cell extract, a cell-free extract, a partially purified protein and a purified protein.

Moreover, GH11 xylanase may be added to the biomass or hemicellulose containing material in the form of a solution or as a solid - depending on the use and/or the mode of application and/or the mode of administration. The solid form can be either as a dried enzyme powder or as a granulated enzyme.

In one embodiment, the invention provides a GH11 xylanase composition for addition to biomass or hemicellulose containing material, said composition comprising a GH11 xylanase according to the invention, and optionally at least one formulating agent, excipient, stabilizer and/or a preservative. The formulation can be a liquid formulation, such as a solution or suspension, or a dry formulation, such as a powder or granulate.

In one embodiment, the GH11 xylanase composition comprises a sugar as heat stabilizing agents e.g., sucrose, trehalose, sorbose, melezitose, verbascose, melibiose, sucralose or raffinose. The sugar provides enhanced thermal stability to the enzymes by encapsulating the enzymes in a sugar matrix so that the activity of the enzymes is maintained at a high level through processing operations.

Liquid enzyme formulations contain a solvent e.g. selected from the group comprising glycerol or water.

In one embodiment, the enzyme composition comprises a stabilizer. Stabilizers may, without being limited to these examples, be selected from:

- salts such as sodium chloride, magnesium chloride, sodium sulfate and potassium sulfate,
- small solutes like ectoine,
- amino acids or proteins, such as histidine, glycine, arginine or BSA,
- polyols, polymers and (poly)saccharides, e.g. starch, oligosaccharides, maltodextrin, trehalose, lactose, maltose, cellodextrins sucrose, mannitol, sorbitol, dextran or PEG;
- surfactants such as gelatin, poloxamers Brij, octyl-glucopyranoside, palmitic acid, dipalmitylphosphatidylcholine, hydroxypropyl-beta-cyclodextrin, polysorbate 20 or polysorbate 80,
- antioxidantia, such as DTT, EDTA, THPP and mercaptoethanol,

- polycations, such as polyethyleneimine, and
- polyanions such as polyacrylic acid.

In a further embodiment, the enzyme composition comprises a preservative. The preservative is e.g. methyl paraben, propyl paraben, benzoate, sorbate or other food approved or non-food approved preservatives or a mixture thereof.

In yet a further embodiment, the enzyme composition comprises at least one other agent selected from the group of additives such as extenders, fillers, binders, flavor maskers, bitter blockers and activity enhancers.

In a further embodiment, the GH11 enzyme according to the invention can be used in combination with one or more accessory enzymes for further improving the xylanase effect. Non-exclusive examples for accessory enzymes are selected from the group comprising amylases, pullulanases, glucoamylases, maltogenic amylases, amyloglucosidase, maltotetraohydrolases, proteinases, other xylanases, acetyltransferases, arabinofuranosidases, beta-xylosidases, beta-mannosidases, fucosidases, rhamnosidases, xylan esterases, glucuronosidases, glucose oxidases, lytic polysaccharide monoxygenases (LPMOs), oxidoreductases, lichenases, lipases, laminarinases, glucanases, cellulases, mannanases, glucomannanases, galactanases, chitosanases, carragenanases, agarases, arabanases, xyloglucanases, fructanases, transglutaminases, isomerases, lipases, phospholipases, phytases, amylases, lipooxygenases, pectinases, rhamnogalacturonan lyases, galacturonyl hydrolases, proteases, peptidases, galacturanases, pectin lyases or a mixture thereof. These accessory enzymes may added together with the GH11 enzyme of the invention to the biomass or hemicellulose containing material or may be comprised in the composition comprising the GH11 enzyme as described hereinabove. It is also possible to add two separate compositions, a first composition comprising the GH11 enzyme of the invention, and a second composition comprising one or more accessory enzymes, to the biomass or hemicellulose containing material.

**Modification of GH11 xylanase**

The GH11 xylanase used in the methods of the invention is obtained from *Clostridium stercorarium.* In 1990, Schwarz et al. published the analysis of xylan degrading xylanases from *Clostridium stercorarium* and Adelsberger et al. described 2004 the recombinant expression of xylanases derived from *Clostridium stercorarium.* In a preferred embodiment, the GH11 xylanase used in the methods of the invention is modified by deleting at least two, preferably all three CBM modules of the parent enzyme using genetic engineering.

In another preferred embodiment the enzyme profile of the GH11 polypeptides according to SEQ ID NOs:1-3 is improved by genetic engineering. An improved enzyme profile includes in one embodiment a further improved thermostability and/or resistance against acid or alkaline treatment. An increased activity profile regarding the enzyme activity at temperatures higher than 85 °C can e.g. be achieved by mutagenesis of enzymes with a suitable product distribution. The person skilled in the art knows the general techniques of introducing mutations into enzymes in order to optimize the enzyme characteristics. Example mutations are the introduction of cysteine residues into the amino acid sequence, which form a disulfide bridge to stabilize the enzyme against thermal denaturation. In another aspect, thermal stability or improved resistance against acid or alkaline treatment could be achieved by random, targeted mutagenesis or directed evolution, whereby one or several amino acids of the original amino acid sequence are substituted by amino acids differing from the original sequence. In another aspect, deletion or insertion of one or more amino acids, loop regions or protein modules in the original amino acid sequence could be performed in order to increase the thermal stability of the enzyme. In another aspect, thermostabilization of the enzyme may be achieved by encapsulation, chemical cross linking of the enzyme and addition of stabilizing compounds. Such stabilizing compounds are for example BSA, glycerol and sorbitol. Other methods to stabilize enzymes is chemical cross-linking or any other method, which leads to a suitable enzyme activity above, equal to or above 85 °C.

The strain of *Clostridium stercorarium* is available to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

In a preferred embodiment, the enzyme used in the methods of the present invention has xylanase activity. More preferably, the enzyme used in the methods of the present invention modifies the content of hemicellulose components. The enzyme alters the xylan content to overcome the limitations of biomass or hemicellulose containing material e.g. compact structure or high viscosity. Altering the polymeric xylan content means in one embodiment the reduction of the content of polymeric xylan. In another embodiment altering the polymeric xylan content means the reduction of the content of polymeric arabinoxylan. In another embodiment, altering the polymeric arabinoxylan content means changing the ratio from insoluble (WU-AX) to soluble (WE-AX) arabinoxylan.

The present invention further provides polypeptides, which have the deduced amino acid sequence of SEQ ID NOs: 1 to 3, as well as fragments, analogs and derivatives of such polypeptides. The terms "fragment", "derivative" and "analog", when referring to a polypeptide of SEQ ID NOs: 1 to 3, means polypeptides that retain essentially the same biological function or activity as a xylanase. An analog might, for example, include a proprotein, which can be activated by cleavage of the proprotein to produce an active mature protein.

The polypeptides of the present invention may be recombinant polypeptides, natural polypeptides, synthetic polypeptides, produced by proteolysis polypeptide or genetically engineered polypeptides. The fragment, derivative or analog of a polypeptide of SEQ ID NOs: 3, may be (i) one in which one or more of the amino acid residues is substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which additional amino acids are fused to the mature protein, such as a leader or secretory sequence or a sequence which is employed for purification, or for substrate or complex binding of the mature polypeptide, or a proprotein sequence. Such fragments, derivatives and analogs are deemed to be within the scope of those skilled in the art to provide upon the basis of the teachings herein.

The polypeptides of the present invention include the polypeptides of SEQ ID NO: 3, as well as polypeptides which have at least 75 % similarity (e.g. preferably at least 50 %; and more preferably at least 70 % identity) to a polypeptide of SEQ ID NO: 3, more preferably at least 85 % similarity (e.g. preferably at least 70 % identity) to a polypeptide of SEQ ID NO: 3, and most preferably at least 95 % similarity (e.g. preferably at least 90 % identity) to a polypeptide of SEQ ID NO: 3. Moreover, they should preferably include exact portions of such polypeptides containing a sequence of at least 30 amino acids, and more preferably at least 50 amino acids.

Fragments or portions of the polypeptides of the present invention may be employed as intermediates for producing the corresponding full-length polypeptides by peptide synthesis. Fragments or portions of the polynucleotides of the present invention may also be used to synthesize full-length polynucleotides of the present invention.

In a preferred embodiment, said polypeptide of the invention is a GH11 xylanase comprising, essentially consisting of or consisting of a polypeptide which has at least 75 % amino acid sequence identity to a polypeptide selected from SEQ ID NOs: 1 to 3 and which shows xylanase activity, wherein the polypeptide of

SEQ ID NO: 1: is the wildtype enzyme from *Clostridium stercorarium*;
SEQ ID NO: 2: is the *Clostridium stercorarium* GH11 xylanase w/o two carbohydrate binding modules (CBMs)
SEQ ID NO: 3: is the *Clostridium stercorarium* GH xylanase w/o three carbohydrate binding modules.

In a more preferred embodiment, said GH11 xylanase with xylanase activity comprises, essentially consists of or consists of a polypeptide which has at least 75 % amino acid sequence identity to the polypeptide according to SEQ ID NO: 2, with the proviso that the enzyme is not the polypeptide of SEQ ID NO: 1.

In a still more preferred embodiment, said GH11 xylanase comprises, essentially consists of or consists of a polypeptide having at least 75 % amino acid sequence identity to a polypeptide of SEQ ID NO: 2.

In a yet more preferred embodiment, said GH11 xylanase comprises, essentially consists of or consists of a polypeptide having at least 75 % amino acid sequence identity to a polypeptide of SEQ ID NO: 2, wherein in said xylanase two CBMs are deleted.

In a most preferred embodiment, said GH11 xylanase with xylanase activity comprises, essentially consists of or consists of a polypeptide which has at least 75 % amino acid sequence identity to the polypeptide according to SEQ ID NO: 3, with the proviso that the enzyme is not the polypeptide of SEQ ID NO: 1.

In a still most preferred embodiment, said GH11 xylanase comprises, essentially consists of or consists of a polypeptide having at least 75 % amino acid sequence identity to a polypeptide of SEQ ID NO: 3.

In a particularly preferred embodiment, said GH11 xylanase comprises, essentially consists of or consists of a polypeptide having at least 75 % amino acid sequence identity to a polypeptide of SEQ ID NO: 3, wherein in said xylanase three CBMs are deleted which results in an 1.6fold enzyme activity increase in combination with an improved thermostability of 10 °C or more and improved stability against acid treatment.

Preferred according to the invention is a GH11 xylanase of any of SEQ ID NOs: 1 to 3, which displays at least 40 % enzyme activity compared to 100 % enzyme activity at its temperature and pH optimum, in particular xylanase activity, at a broad pH range preferably in the range of 5.0 to 9.5.

Preferred according to the invention is a GH11 xylanase of any of SEQ ID NOs: 1 to 3, which displays at least 40 % enzyme activity compared to 100 % enzyme activity at its temperature and pH optimum, in particular xylanase activity at a broad temperature range from 37 °C to equal to or over 80 °C.

Preferred according to the invention is a GH11 xylanase of any SEQ ID NOs: 1 to 3 which displays enzyme activity, in particular xylanase activity, over a broad range of temperatures in the range from 37 °C to equal to or over 80 °C. The temperature range is important for the broad use of the enzyme in different applications such as animal feed, brewery, pulp and paper and bioenergy.

The GH11 xylanase of the invention is especially suitable for use in improving the conversion of biomass or conversion of hemicellulose containing material, e.g. for use as a digestive improver in animal feed pellets, reduction of viscosity and clearance in brewery, viscosity reduction in ethanol production based on cereals, viscosity reduction in lignocellulose hydrolysis, removal of hemicellulose in pulp and paper. The enzyme provided by the invention still displays a sufficient xylanase activity to modify the arabinoxylan content or chain length at moderate and high temperatures. The stability in alkaline and acidic pH ranges is an advantage for maintaining enzyme activity after subjecting the GH11 xylanase of the invention to severe treatment conditions.

In a preferred embodiment, the invention therefore relates to the use of the GH11 enzyme of the invention or the composition comprising said GH11 enzyme in the production of animal feed, pulp and paper, bioenergy and in the brewery.

The invention further relates to a nucleic acid molecule comprising a nucleic acid sequence encoding the GH10 enzyme of the polypeptides of SEQ ID NOs: 1 to 3 according to the invention, in particular encoding an amino acid sequence selected from SEQ ID NO: 2 or 3. In a preferred embodiment the invention provides a nucleic acid molecule comprising a nucleic acid sequence selected from SEQ ID NOs: 8 to 10, more preferably of SEQ ID NOs:. 9 or 10.

The nucleic acid molecule of SEQ ID NO: 8 encodes the GH xylanase of SEQ ID NO: 1. The nucleic acid molecule of SEQ ID NO: 9 encodes the GH xylanase of SEQ ID NO: 2. The nucleic acid molecule of SEQ ID NO: 10 encodes the GH xylanase of SEQ ID NO: 3.

The "polynucleotides" or "nucleic acids" of the present invention may be in the form of RNA or in the form of DNA; DNA should be understood to include cDNA, genomic DNA, recombinant DNA and synthetic DNA. The DNA may be double-stranded or single-stranded and, if single stranded, may be the coding strand or non-coding (antisense) strand. The coding sequence, which encodes the polypeptide may be identical to the coding sequence for the polypeptides shown in SEQ ID NOs: 1 to 3, preferably of SEQ ID NO: 3, or it may be a different coding sequence encoding the same polypeptide, as a result of the redundancy or degeneracy of the genetic code or a single nucleotide polymorphism. For example, it may also be an RNA transcript which includes the entire length of coding sequence for a polypeptide of any one of SEQ ID NO: 3. In a preferred embodiment, the "polynucleotide" according to the invention is one of SEQ ID NO: 10.

The nucleic acids which encode the polypeptides of SEQ ID NOs: 1 to 3, preferably of SEQ ID NO: 3 may include but are not limited to the coding sequence for the polypeptide alone; the coding sequence for the polypeptide plus additional coding sequence, such as a leader or secretory sequence or a proprotein sequence; and the coding sequence for the polypeptide (and optionally additional coding sequence) plus non-coding sequence, such as introns or a non-coding sequence 5' and/or 3' of the coding sequence for the polypeptide.

Thus, the term "polynucleotide encoding a polypeptide" or the term "nucleic acid encoding a polypeptide" should be understood to encompass a polynucleotide or nucleic acid which includes only a coding sequence for a GH11 xylanase of the invention, e.g. a polypeptide selected from SEQ ID NOs: 1 to 3, preferably of SEQ ID NO: 3 as well as one which includes additional coding and/or non-coding sequence. The terms polynucleotides and nucleic acid are used interchangeably.

The present invention also includes polynucleotides where the coding sequence for the polypeptide may be fused in the same reading frame to a polynucleotide sequence which aids in expression and secretion of a polypeptide from a host cell; for example, a leader sequence which functions as a secretory sequence for controlling transport of a polypeptide from the cell may be so fused. The polypeptide having such a leader sequence is termed a preprotein or a preproprotein and may have the leader sequence cleaved by the host cell to form the mature form of the protein. These polynucleotides may have a 5' extended region so that it encodes a proprotein, which is the mature protein plus additional amino acid residues at the N-terminus. The expression product having such a prosequence is termed a proprotein, which is an inactive form of the mature protein; however, once the prosequence is cleaved, an active mature protein remains. The additional sequence may also be attached to the protein and be part of the mature protein. Thus, for example, the polynucleotides of the present invention may encode polypeptides, or proteins having a prosequence, or proteins having both a prosequence and a presequence (such as a leader sequence).

The polynucleotides of the present invention may also have the coding sequence fused in frame to a marker sequence, which allows for purification of the polypeptides of the present invention. The marker sequence may be an affinity tag or an epitope tag such as a polyhistidine tag, a streptavidin tag, a Xpress tag, a FLAG tag, a cellulose or chitin binding tag, a glutathione-S transferase tag (GST), a hemagglutinin (HA) tag, a c-myc tag or a V5 tag.

The HA tag would correspond to an epitope obtained from the influenza hemagglutinin protein (Wilson et al., 1984), and the c-myc tag may be an epitope from human Myc protein (Evans et al., 1985).

The present invention is considered to further provide polynucleotides which hybridize to the hereinabove-described sequences wherein there is at least 70 %, preferably at least 90 %, and more preferably at least 95 % identity or similarity between the sequences, and thus encode proteins having similar biological activity. Moreover, as known in the art, there is "similarity" between two polypeptides when the amino acid sequences contain the same or conserved amino acid substitutes for each individual residue in the sequence. Identity and similarity may be measured using sequence analysis software (e.g., ClustalW at PBIL (Pôle Bioinformatique Lyonnais) http://npsa-pbil.ibcp.fr). The present invention particularly provides such polynucleotides, which hybridize under stringent conditions to the hereinabove-described polynucleotides.

Suitably stringent conditions can be defined by, e.g., the concentration of salt or formamide in the prehybridization and hybridization solution, or by the hybridization temperature, and are well known in the art. In particular, stringency can be increased by reducing the concentration of salt, by increasing the concentration of formamide, and/or by raising the hybridization temperature.

For example, hybridization under high stringency conditions may employ about 50 % formamide at about 37 °C to 42 °C, whereas hybridization under reduced stringency conditions might employ about 35 % to 25 % formamide at about 30 °C to 35 °C. One particular set of conditions for hybridization under high stringency conditions employs 42 °C, 50 % formamide, 5x SSPE, 0.3 % SDS, and 200 μg/ml sheared and denatured salmon sperm DNA. For hybridization under reduced stringency, similar conditions as described above may be used in 35 % formamide at a reduced temperature of 35 °C. The temperature range corresponding to a particular level of stringency can be further narrowed by calculating the purine to pyrimidine ratio of the nucleic acid of interest and adjusting the temperature accordingly. Variations on the above ranges and conditions are well known in the art. Preferably, hybridization should occur only if there is at least 95 %, and more preferably at least 97 %, identity between the sequences. The polynucleotides which hybridize to the hereinabove described polynucleotides in a preferred embodiment encode polypeptides which exhibit substantially the same biological function or activity as the mature protein of SEQ ID NOs: 1 to 3, preferably of SEQ ID NO: 3.

As mentioned, a suitable polynucleotide probe may have at least 14 bases, preferably 30 bases, and more preferably at least 50 bases, and will hybridize to a polynucleotide of the present invention, which has an identity thereto, as hereinabove described. For example, such polynucleotides may be employed as a probe for hybridizing to the polynucleotides encoding the polypeptides of SEQ ID NO: 3, such as the polynucleotides of SEQ ID NO: 10, respectively, for example, for recovery of such a polynucleotide, or as a diagnostic probe, or as a PCR primer. Thus, the present invention includes polynucleotides having at least a 70 % identity, preferably at least a 90 % identity, and more preferably at least a 95 % identity to a polynucleotide of SEQ ID NO: 10, which encodes a polypeptide of SEQ ID NO: 3, as well as fragments thereof, which fragments preferably have at least 30 bases and more preferably at least 50 bases.

The terms "homology" or "identity," as used interchangeably herein, refer to sequence similarity between two polynucleotide sequences or between two polypeptide sequences, with identity being a more strict comparison. The phrases "percent identity or homology" and "identity or homology" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100 % similar, or any integer value there between. Identity or similarity can be determined by comparing a position in each sequence that can be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences.

A degree of identity of polypeptide sequences is a function of the number of identical amino acids at positions shared by the polypeptide sequences. A degree of homology or similarity of polypeptide sequences is a function of the number of amino acids at positions shared by the polypeptide sequences. The term "substantially identical," as used herein, refers to an identity or homology of at least 70 %, 75 %, at least 80 %, at least 85 %, at least 90 %, 91 %, 92 %, 93 %, 94 %, 95 %, 96 %, 97 %, 98 %, 99 %, or more.

The degree of sequence identity is determined by choosing one sequence as the query sequence and aligning it with the internet-based tool ClustalW with homologous sequences taken from GenBank using the blastp algorithm (NCBI).

As it is well known in the art, the genetic code is redundant in that certain amino acids are coded for by more than one nucleotide triplet (codon), and the invention includes those polynucleotide sequences which encode the same amino acids using a different codon from that specifically exemplified in the sequences herein. Such a polynucleotide sequence is referred to herein as an "equivalent" polynucleotide sequence. The present invention further includes variants of the hereinabove described polynucleotides which encode for fragments, such as part or all of the protein, analogs and derivatives of a polypeptide of SEQ ID NO: 3. The variant forms of the polynucleotide may be a naturally occurring allelic variant of the polynucleotide or a non-naturally occurring variant of the polynucleotide. For example, the variant in the nucleic acid may simply be a difference in codon sequence for the amino acid resulting from the degeneracy of the genetic code, or there may be deletion variants, substitution variants and addition or insertion variants. As known in the art, an allelic variant is an alternative form of a polynucleotide sequence, which may have a substitution, deletion or addition of one or more nucleotides that does not substantially alter the biological function of the encoded polypeptide.

The present invention also includes vectors, which include such polynucleotides, host cells, which are genetically engineered with such vectors, and the production of the polypeptides of SEQ ID NOs: 1 to 3, preferably of SEQ ID NO: 3 by recombinant techniques using the foregoing. Host cells are genetically engineered (transduced or transformed or transconjugated or transfected) with such vectors, which may be, for example, a cloning vector or an expression vector. The vector may be, for example, in the form of a plasmid, a conjugative plasmid, a viral particle, a phage, etc. The vector or the gene may be integrated into the chromosome at a specific or a not specific site. Methods for genome integration of recombinant DNA, such as homologous recombination or transposase-mediated integration, are well known in the art. The engineered host cells can be cultured in conventional nutrient media modified as appropriate for activating promoters, selecting transformants or amplifying the genes of the present invention. The culture conditions, such as temperature, pH and the like, are those commonly used with the host cell selected for expression, as well known to the ordinarily skilled artisan.

**Expression**

The polynucleotides of the present invention may be employed for producing the polypeptides of SEQ ID NOs: 1 to 3, preferably of SEQ ID NO: 2 or 3, most preferably of SEQ ID NO: 3, by recombinant techniques. Thus, for example, the polynucleotides may be included in any one of a variety of expression vectors.

The appropriate DNA sequence may be inserted into the vector by any of a variety of procedures. In general, the DNA sequence is inserted into an appropriate restriction endonuclease site(s) by procedures well known in the art, which procedures are deemed to be within the scope of those skilled in this art.

The DNA sequence in the expression vector is operatively linked to an appropriate expression control sequence(s) (promoter) to direct mRNA synthesis. As representative examples of such promoters, there may be mentioned: LTR or SV40 promoter, the *E. coli lac, ara, rha* or *trp,* the phage lambda $P_L$ promoter and other promoters known to control expression of genes in prokaryotic or eukaryotic cells or their viruses.

More preferably, the GH11 xylanase of the invention can be expressed using the following tools:

> Specific examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*), *Bacillus amyloliquefaciens* alpha-amylase gene (*amyQ*), *Bacillus licheniformis* penicillinase gene (*penP*), *Bacillus subtilis* xylose based expression via *xylA* and *xylB* genes, *B. subtilis* $sigma^B$ dependent expression of general stress proteins (*gsiB*), and prokaryotic beta-lactamase gene (Villa-Kamaroff et al., 1978), as well as the tac promoter (DeBoer et al., 1983). Furthermore, the constitutive promotors p43 of *B. subtilis* and hpall of *Staphylococcus aureus.* Further promoters are described in "Useful proteins from recombinant bacteria" in Scientific American, 1980, 242: 74-94; and in Sambrook et al., 1989. Also possible are hybrids, and double or triple combinations of the above mentioned promotors, as well as mutated and truncated variants thereof.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei xylanase* II, *Trichoderma reesei beta-xylosidase,* as well as the NA2-tpi promoter (a modified promoter from the gene encoding neutral alpha-amylase in *Aspergillus niger* in which the untranslated leader has been replaced by an untranslated leader from the gene encoding triose phosphate isomerase in *Aspergillus nidulans*); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992. In a *Pichia* host, useful promoters are obtained from the genes for *Pichia pastoris* alcohol oxidase (AOX1) and *Pichia pastoris* glyceraldehyde 3-phosphate dehodrogenase (GAP).

The control sequence may also be a suitable transcription terminator sequence, a sequence recognized by a host cell to terminate transcription. The terminator sequence is operably linked to the 3' terminus of the nucleotide sequence encoding the polypeptide. Any terminator that is functional in the host cell of choice may be used in the present invention. Preferred terminator structures are for example from the *Bacillus amyloliquefaciens amyE* gene, the *Bacillus licheniformis penP* gene, the *B. subtilis bglS* or *apreE* gene, and the *Bacillus thuringiensis cry* gene.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992.

The control sequence may also be a suitable leader sequence, a nontranslated region of an mRNA that is important for translation by the host cell. The leader sequence is operably linked to the 5' terminus of the nucleotide sequence encoding the polypeptide. Any leader sequence that is functional in the host cell of choice may be used in the present invention. Preferred nontranslated regions are from the *Bacillus amyloliquefaciens amyE* gene, the *Bacillus licheniformis penP* gene, the *B. subtilis bglS* or *apreE* gene, and the *Bacillus thuringiensis cry* gene.

Preferred leader sequences for filamentous fungal host cells are obtained from the genes for *Aspergillus oryzae* TAKA amylase and *Aspergillus nidulans* triose phosphate isomerase.

Suitable leader sequences for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3' terminus of the nucleotide sequence and, when transcribed, is recognized by the host cell as a signal to add polyadenosine residues to transcribed mRNA. Any polyadenylation sequence that is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995.

The control sequence may also be a signal peptide coding sequence that encodes a signal peptide linked to the amino terminus of a polypeptide and directs the encoded polypeptide into the cell's secretory pathway. The 5' end of the coding sequence of the nucleotide sequence may inherently contain a signal peptide coding sequence naturally linked in the translation reading frame with the segment of the coding sequence that encodes the secreted polypeptide. Alternatively, the 5' end of the coding sequence may contain a signal peptide coding sequence that is foreign to the coding sequence. The foreign signal peptide coding sequence may be required where the coding sequence does not naturally contain a signal peptide coding sequence. Alternatively, the foreign signal peptide coding sequence may simply replace the natural signal peptide coding sequence in order to enhance secretion of the polypeptide.

However, any signal peptide coding sequence that directs the expressed polypeptide into the secretory pathway of a host cell of choice, i.e., secreted into a culture medium, may be used in the present invention.

Effective signal peptide coding sequences for bacterial host cells are the signal peptide coding sequences obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT*, *nprS*, *nprM*), and *Bacillus subtilis prsA*. Further signal peptides are described by Simonen and Palva, 1993, and Brockmeier et al., 2006.

Effective signal peptide coding sequences for filamentous fungal host cells are the signal peptide coding sequences obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, *Humicola insolens* endoglucanase V, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, supra.

The control sequence may also be a propeptide coding sequence that encodes a propeptide positioned at the amino terminus of a polypeptide. The resultant polypeptide is known as a proenzyme or propolypeptide (or a zymogen in some cases). A propeptide is generally inactive and can be converted to a mature active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (nprT), *Saccharomyces cerevisiae* alpha-factor, *Rhizomucor miehei* aspartic proteinase, and *Myceliophthora thermophila* laccase (WO 95/33836).

Where both signal peptide and propeptide sequences are present at the amino terminus of a polypeptide, the propeptide sequence is positioned next to the amino terminus of a polypeptide and the signal peptide sequence is positioned next to the amino terminus of the propeptide sequence.

It may also be desirable to add regulatory sequences that allow the regulation of the expression of the polypeptide relative to the growth of the host cell. Examples of regulatory systems are those that cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system, GAL1 system or AOX1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the nucleotide sequence encoding the polypeptide would be operably linked with the regulatory sequence.

**Expression Vectors**

The present invention also relates to recombinant expression vectors comprising a polynucleotide of the present invention, a promoter, and transcriptional and translational stop signals. The various nucleic acids and control sequences described herein may be joined together to produce a recombinant expression vector that may include one or more (several) convenient restriction sites to allow for insertion or substitution of the nucleotide sequence encoding the polypeptide at such sites. Alternatively, a polynucleotide sequence of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vectors of the present invention preferably contain one or more (several) selectable markers that permit easy selection of transformed, transfected, transduced, or the like cells. A selectable marker is a gene the product of which provides for biocide or viral resistance, resistance to heavy metals, prototrophy to auxotrophs, and the like (Kroll et al., 2009). These auxotrophies include but are not limited to disruptions or deletions in amino acid biosynthesis for alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine, respectively. These auxotrophies may also include but are not limited to disruptions or deletions in purine, pyrimidine or enzyme cofactor biosynthesis genes. The auxotrophic phenotype is complemented episomally comprising an intact and expressed version of the mutated gene causing the auxotrophy together with an expression cassette containing the gene of interest.

Examples of bacterial selectable markers are the dal genes from *Bacillus subtilis* or *Bacillus licheniformis,* or markers that confer antibiotic resistance such as ampicillin, kanamycin, chloramphenicol, or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*
The vectors of the present invention preferably contain an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" is defined herein as a nucleotide sequence that enables a plasmid or vector to replicate in vivo.

Examples of bacterial origins of replication are the origins of replication of plasmids pBR322, pUC19, pACYC177, and pACYC184 permitting replication in *E. coli*, and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of the gene product. An increase in the copy number of the polynucleotide can be achieved by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

In a preferred embodiment, the invention provides a host cell expressing the enzyme according to one of SEQ ID NOs: 1 to 3, preferably of SEQ ID NO: 2 or 3, most preferably of SEQ ID NO: 3. In another preferred embodiment, said host cell comprises a nucleotide molecule selected from the polynucleic acid of SEQ ID NOs: 8 to 10, preferably of SEQ ID NO: 9 or 10, most preferably of SEQ ID NO: 10. Most preferably, said host cell is *E. coli* or *Bacillus subtilis.*

## Methods of Production

The present invention provides in a further embodiment a method for producing an enzyme of SEQ ID NOs: 1 to 3, preferably of SEQ ID NO: 2 or 3, most preferably of SEQ ID NO: 3, the method comprising culturing a host cell as described herein under conditions permitting the production of the enzyme, and recovering the enzyme from the culture.

More preferably, the present invention provides methods of producing a polypeptide of the present invention, i.e. an enzyme of SEQ ID NOs: 1 to 3, preferably of SEQ ID NO: 2 or 3, most preferably of SEQ ID NO: 3, comprising: (a) cultivating a cell, which produces the polypeptide, under conditions conducive for production of the polypeptide; and (b) recovering the polypeptide. In a preferred aspect, the cell is of the genus *Bacillus.* In a more preferred aspect, the cell is *Bacillus subtilis or Bacillus licheniformis.*

The present invention also relates to methods of producing a polypeptide of the present invention, comprising: (a) cultivating a recombinant host cell, as described herein, under conditions conducive for production of the polypeptide; and (b) recovering the polypeptide.

The present invention also relates to methods of producing a polypeptide of the present invention, comprising: (a) cultivating a recombinant host cell under conditions conducive for production of the polypeptide, wherein the host cell comprises a gene for an enzyme , more specifically an enzyme obtained from a bacterium, more specifically a recombinant bacterial enzyme according to SEQ ID NO: 3.

In the production methods of the present invention, the cells are cultivated in a nutrient medium suitable for production of the polypeptide using methods well known in the art. For example, the cell may be cultivated by shake flask cultivation, and small-scale or large-scale fermentation (including continuous, batch, fed-batch, or solid-state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted into the medium, it can be recovered from cell lysates.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide may be recovered using methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides.

**Compositions**

The present invention also relates to compositions comprising a polypeptide of the present invention, i.e. an enzyme of SEQ ID NOs: 1 to 3, preferably of SEQ ID NO: 2 or 3, most preferably of SEQ ID NO: 3. Preferably, the compositions are enriched in such a polypeptide. The term "enriched" indicates that the xylanase activity of the composition has been increased, e.g., with an enrichment factor of at least 1.1.

The polypeptide compositions may be prepared in accordance with methods known in the art and may be in the form of a liquid or a dry composition. The polypeptide to be included in the composition may be stabilized in accordance with methods known in the art and as described hereinbefore.

Enzyme Preparation

The invention further provides an enzyme preparation comprising an enzyme of SEQ ID NOs: 1 to 3, preferably of SEQ ID NO: 2 or 3, most preferably of SEQ ID NO: 3, for use for conversion of biomass or conversion of hemicellulose containing material. The enzyme preparation is preferably in the form of a liquid, granulate or agglomerated powder, more preferably in the form of a granulate or agglomerated powder.

The granulate or agglomerated powder has preferably a narrow particle size distribution with more than 95 % (by weight) of the particles in the range from 25 to 500 micrometer.

Granulates and agglomerated powders may be prepared by conventional methods, e.g. by spraying the xylanase onto a carrier in a fluid-bed granulator. The carrier may consist of particulate cores having a suitable particle size. The carrier may be soluble or insoluble, e.g. a salt (such as sodium chloride or sodium sulfate), a sugar (such as sucrose or lactose), a sugar alcohol (such as sorbitol), an oligosaccharide (such as maltodextrin), starch, rice, corn grits, or soy.

**Brief description of the drawings**

Specific embodiments of the present invention are described in the working examples with reference to the accompanying drawings.

Herein,

Figure 1 shows an SDS-PAGE of recombinantly produced enzymes of SEQ ID NOs: 1 to 3 respectively. PageRuler Prestained Protein Ladder 10 to 180 kDa (#26616, ThermoFisher Scientific) was used as protein standard.

Figure 2 shows the relative enzyme activity increase (%) of the polypeptides of SEQ ID NOs: 3 and 2 in relation to the enzyme activity of the wildtype enzyme of SEQ ID NO 1. Surprisingly, the enzyme of SEQ ID NO: 3 displays a 1,6 fold higher activity in comparison to the enzyme activity of the wildtype enzyme of SEQ ID NO: 1. Enzyme activity: the release of the amount (micromole) of reducing sugars from arabinoxylan per mg of enzyme per minute was determined with the DNSA assay compared to the activity of the polypeptide of SEQ ID NO: 1. Enzyme activity was measured in triplicates.

Figure 3 shows the relative enzyme activity of the polypeptide SEQ ID NO: 3 tested under different pH and temperature conditions Enzyme activity is defined as the release of the amount (micromole) of reducing sugars from arabinoxylan per mg of enzyme per minute as was determined with the DNSA assay and compared to the activity at 70 °C and pH 7.0. Enzyme activity was measured in triplicates.

Figure 4 shows the resistance of the polypeptide of SEQ ID NO: 3 against high temperatures simulating the pelleting process. Enzyme activity is defined as the release of the amount (micromole) of reducing sugars from arabinoxylan per mg of enzyme per minute as was determined with the DNSA assay and compared to the activity of the untreated enzyme. The activity was measured in triplicates at 60 °C at pH 6.5.

Figure 5 shows the stability of the polypeptide of SEQ ID NO: 1 and SEQ ID NO: 3 against acid treatment. To simulate the gastric tract *in vitro,* the polypeptides of SEQ ID NO: 1 and SEQ ID NO: 3 were incubated at pH 3.5 at 40 °C for 30, 60 and 120 min. The remaining activity was determined by DNSA assay and compared to the activity of the untreated protein. Enzyme activity is defined as the release of the amount (micromole) of reducing sugars from arabinoxylan per mg of enzyme per minute was determined with the DNSA assay and compared to the activity of the untreated enzyme. The activity was measured in triplicates at pH 6.5 at 70 °C.

Figure 6 shows the residual activity of the polypeptide given in SEQ ID NO: 3 against proteolytic digestion by pepsin at 40 °C and pH 3.5. Residual activity was determined by measuring the release of reducing sugar equivalents (micromole per minute per mg enzyme) on arabinoxylan at 60 °C and pH 6.5 using the DNSA assay.

Figure 7 shows the reduction of viscosity by the polypeptide of SEQ ID NO: 3 in comparison to Danisco Xylanase in an *in vitro* chicken intestine model.

Figure 8 shows the time for liquefaction of wheat slurry without enzyme or with addition of different enzymes: A: alpha-amylase 25 mg/kg of Teramyl 3000L (Novozymes), B: alpha-amylase 25 mg/kg and SEQ ID NO 3, C: alpha-amylase 25 mg/kg, SEQ ID NO 3 and an endoglucanase SEQ ID NO 11 (EP17203087), D: alpha-amylase 25 mg/kg) cellulase/hemicellulase mixture Cellic CTec2 (Novozymes). Without an alpha-amylase no liquefaction was observed (asterisk). For SEQ ID NOs: 3 and 11 as well as Cellic CTec2 equal amounts of protein were used (0.4 mg/kg). All measurements were done in triplicate.

**Working Examples**

**Example 1: Cloning of the polynucleic acids according to SEQ ID NOs: 8 to 10 encoding the GH11 xylanases of SEQ ID NOs: 1-3**

*Materials*

Chemicals used as buffers and substrates were commercial products of at least reagent grade. *Escherichia coli* DH10B was used for routine cloning and *E. coli* BL21 Star (DE3) for expression of *Clostridium stercorarium* DSM8532 of SEQ ID NO 8.

*DNA modification*

Preparation of chromosomal and plasmid DNA, endonuclease digestion, and ligation were carried out by standard procedures (Sambrook J and Russell DW. 2001).

*Cloning of genes encoding GH11 xylanases*

The genes with sequences of SEQ ID NOs: 8 to 10 were amplified from chromosomal DNA from *C. stercorarium* DSM8532 in accordance with manufacturer's instructions (Phusion High-Fidelity DNA Polymerase, F530S, ThermoFisher Scientific) using primer set of SEQ ID NOs: 4 and 5. With the GH 11 xylanases of SEQ ID NO: 2 and 3, the effect of different protein module (i.e. CBM) deletions on enzyme activity and function was investigated. Two carbohydrate binding domains were deleted in the polypeptide of SEQ ID NO: 2 enzyme and all three carbohydrate binding modules were deleted in the polypeptide of SEQ ID NO: 3. The polynucleic acid of SEQ ID NO: 9 was amplified using primer 4 and 6, and the polynucleic acid of SEQ ID NO: 10 was amplified by employing primer 4 and 7. All PCR products were subsequently cloned by Gibson assembly (NEB, Cat. Nr. E2611S) in *Nde*I/*Xho*I digested pET24c(+) vector (Novagen, MerckMillipore) and sequenced by Eurofins to confirm the correct sequence.

**Example 2: Protein production of the enzymes of SEQ ID NOs 1 to 3**

*Growth of cells*

Fed-batch fermentations of recombinant *E. coli* strains harbouring the GH11 Xylanase genes from *C. stercorarium* DSM8532 of SEQ ID NO: 8 and the newly designed genes SEQ ID Nos: 9 and 10 were carried out in a 10 L Uni-Vessel controlled and equipped with a Biostat B Twin DCU (Sartorius AG, Göttingen, Germany). Temperature, pH, foam, turbidity, weight and dissolved oxygen were monitored online during fermentation. The dissolved oxygen (DO%) was set to 25 % (vol/vol) and maintained with increasing agitation at constant air flow. The formation of foam was controlled by the addition of Antifoam 206 (Sigma Aldrich, St. Louis, Missouri, USA). A pH of 6.9 was maintained by addition of a 25 % (vol/vol) ammonium hydroxide solution or 25 % (vol/vol) $HPO_4$ solution. *E. coli* strains were cultivated in Riesenberg medium (Korz et al., 1995) at the 10 L scale, the feeding solution consists of 1021 g/L glycerol, 20 g/L $MgSO_4 \cdot 7\ H_2O$, 13 mg/L EDTA, 4 mg/L $CoCl_2 \cdot 6\ H_2O$, 23.5 mg/L $MnCl_2 \cdot 4\ H_2O$, 2.5 mg/L $CuC1_2 \cdot 2\ H_2O$, 5 mg/L

$H_3BO_3$, 4 mg/L $Na_2MoO_4$ x 2 $H_2O$, 16 mg/L $Zn(CH_3COO)_2$ • 2 $H_2O$, 40 mg/L Fe(III)citrate (Korz et al., 1995). After the consumption of the initial carbohydrate substrate, growth rate was controlled according to EQUATION 1, whereby ms, is the mass flow of substrate (g $h^{-l}$), $\mu_{set}$ the desired specific growth rate ($h^{-l}$), $Y_{X/S}$ the biomass/substrate yield coefficient (g $g^{-1}$), m the specific maintenance coefficient (g $g^{-1}$ $h^{-l}$), V the cultivation volume (L), and X the biomass concentration (g $L^{-1}$):

EQUATION 1

$$m_S = \left( \frac{\mu_{set}}{Y_{X/S}} + m \right) \cdot V(t) \cdot X(t) \cdot e^{\mu_{set}(t-t_F)}$$

The inoculation procedure was the following: Based on a cryo-stock, a fresh agar plate containing adequate antibiotics was prepared. With one colony an Erlenmeyer flask containing 30 mL Lysogeny Broth (Sambrook et al. 1989) was inoculated and incubated for 12 to 15 h at 30 °C. 30 mL of this first preculture was used to inoculate 500 mL of the fermentation medium in a 5 L Erlenmeyer flask and incubated for further 14 h. The 10 L fermenter was filled with 6 L fermentation medium and inoculated with 500 mL of the second preculture. Kanamycin was added at 50 $\mu$g/mL. Protein production was induced by changing the glycerol feed to lactose feed. Cells were harvested after 48 h by centrifugation for 1 h at 9000 rpm and 22 °C. Portions of 300 g cells were solved in 3 L lysis buffer (50 mM MOPS pH 7.3, 0.1 M NaCl, 20 mM imidazol). Cell lysis was achieved by ultrasonic treatment in an ultrasonic flow-through chamber. Cell debris was separated by centrifugation (9000 rpm, 22 °C). Supernatant was clarified from residual cells or debris by tangential filtration applying a 0.2 $\mu$m filter cassette and three volumes washing with lysis buffer. The enzyme solution was concentrated employing tangential filtration with a 30 kDa filter cassette followed by dialysis with three volumes lysis buffer. GH11 xylanases were purified by immobilized metal ion affinity chromatography (IMAC). Pure enzymes were eluted with elution buffer containing 50 mM MOPS, pH 7.3, 0.25 M imidazole, 0.1 M NaCl, and 20 mM $CaCl_2$.

Another possibility for producing variants of the SEQ ID NO: 1 enzyme, such as the enzymes of SEQ ID NOs: 2 or 3 of the invention is to transform a competent *B. subtilis* strain with an appropriate vector comprising the mutated DNA and cultivating the recombinant strain in accordance with Park et al., 1991.

**Example 3: Electrophoretic methods**

Sodium dodecyl sulphate (SDS) polyacrylamide gel electrophoresis was performed in accordance with Laemmli (1970). Proteins were resuspended in denaturating buffer and heated for 15 min at 95 °C. The PageRuler Prestained Protein Ladder 10 to 180 kDa (#26616, ThermoFischer Scientific) was used as molecular weight standard. The proteins were stained with Coomassie brilliant blue R-250 (Weber and Osbourne, 1969).

**Example 4: Protein Quantification**

The protein amount was determined by using Pierce™ BCA Protein Assay Kit (#23225, ThermoFisher Scientific) in accordance with the instructions of the manufacturer.

**Example 5: Activity test, Arabinoxylan degradation**

*Xylanase activity*

For the purpose of the present invention, any of the commercially available xylanase activity measurement kits is suitable to determine xylanase activity. One suitable way of measuring the xylanase activity is as follows:

> Xylanase activity measurement was with a final concentration of 0.86 % (wt/vol) arabinoxylan (wheat arabinoxylan, medium viscosity, Megazyme, Ireland), reaction buffer (100 mM MOPS, pH 6.5, 50 mM NaCl, 10 mM $CaCl_2$) and the appropriate amount of enzyme. Quantification of reducing sugars was performed as described by Wood and Bhat (1988) using 3,5-dinitrosalicylic acid (DNSA). The amount of liberated reducing sugar ends was determined based on a calibration curve with glucose. One unit (U) is defined as the amount of enzyme required to liberate one $\mu$mole reducing sugar equivalents in one minute. All assays were performed at least in triplicates.

The *Clostridium stercorarium* xylanase of SEQ ID NO: 1 and the variants of SEQ ID NOs: 2 and 3 were produced, purified and quantified as described in Examples 1-4. The specific activity of the enzymes of SEQ ID NOs: 1 to 3 was calculated as U/mmol. The results were calculated as ratio to the parent enzyme SEQ ID NO: 1. Figure 1 shows the size of the enzymes of SEQ ID NOs: 2 and 3 compared to the size of the parent protein of SEQ ID NO: 1. The variants enzymes SEQ ID NOs: 2 and 3 have a reduced size of 57% and 35% respectively compared to the parent enzyme SEQ ID NO: 1 (Figure 1). The calculation of the specific enzyme activity in respect to the molar mass of the individual enzymes revealed that the specific enzyme activity of SEQ ID NO: 2 compared to that of SEQ ID NO: 1 is reflected by the size difference perfectly. However, surprisingly the variant of SEQ ID NO: 3 displays an at least 1.6 fold specific enzyme activity of compared to the specific enzyme activity of the polypeptides of SEQ ID NOs: 1 and 2 (Figure 2), which does not correspond to the size changes (Figure 1). Surprisingly, the deletion of all carbohydrate binding modules leads to an increase of enzyme activity.

The activity assay was used to define the activity profile of SEQ ID NO: 3 over a broad range of temperatures (25 °C to 90 °C) and pH 4.0-10.0. The enzyme activity measured at 70 °C and pH 7.0 was set to 100%. We identified a sufficient enzyme activity (40% or higher) from 35 °C to 85 °C in a broad pH range from pH 5.0 to 9.5 (Figure 3) indicating a suitable profile for different industrial applications.

**Example 6: Thermostability ($Tm_{on}$ and $Tm_{50}$) of the polypeptides of SEQ ID NOs: 1-3**

To determine the physical stability of the SEQ ID NOs 1-3 variants, differential scanning fluorimetry (DSF) was applied. Proteins were diluted for DSF to a concentration of 0.1 mg/ml in 20 μl 50 mM K-phosphate buffer (pH 7.0, 50 mM NaCl and 5x Sypro™ Orange (Thermo Fisher Scientific)). Measurements were performed in a Bio-Rad Real-Time PCR CF X96 Touch™ Real-Time PCR Detection System. For this purpose the samples were equilibrated for 3 min at 25 °C and then heated with a ramp of 2 °C/min to 98 °C. The emitted fluorescence was detected in the HEX channel (Ex.: 525 ± 10 nm; Em.: 575 ± 15 nm). To determine the $Tm_{50}$, experimental data were analyzed by non-linear regression via the Boltzmann equation as described by Niesen et al. (2007) using GraphPad Prism v6.

$$y = LL + \frac{UL - LL}{1 + \frac{\exp(Tm_{50} - X)}{slope}}$$

The $Tm_{50}$ describes the temperature of 50 % protein denaturation. This corresponds to the X-axis intercept with maximal slope. The onset of protein denaturation, when 1 % of the total protein is denatured is described by $Tm_{on}$ and was determined as described by Menzen and Friess (2013) with values obtained from the Boltzmann equation.

**Table 1: Determination of the melting point of the polypeptides SEQ ID NOs: 1-3**

| | (SEQ ID NO: 1) | (SEQ ID NO: 2) | (SEQ ID NO: 3) |
|---|---|---|---|
| $TM_{50}$ [°C] | 82,9 ±0,57 | 82,8 ±0,8 | 85,7 ±0,21 |
| $Tm_{on}$ [°C] | 67,0 ±0,04 | 63,5 ±0,25 | 81,2 ±0,27 |

The $Tm_{50}$ and $Tm_{on}$ both reflect the thermostability of an enzyme. With the deletion of all carbohydrate binding modules, the $Tm_{on}$ of the polypeptide of SEQ ID NO: 3 was increased to 81.2 °C, which is an increase of more than 10 °C compared to the wildtype enzyme of SEQ ID NO: 1. In addition an increase of $Tm_{50}$ to 85.7 °C could be achieved by the deletion of all CBMs making SEQ ID NO: 3 more thermostable than SEQ ID NO: 1.

**Example 7: Thermostability (TM) of the polypeptides of SEQ ID NO: 3**

By indirectly measuring thermostability enzyme inactivation can be measured as function of temperature. Here enzyme samples are incubated without substrate for a defined period of time e.g. 5 min at various temperatures and following incubation assayed for residual activity at the permissive temperature. Residual activity at each temperature is calculated as relative to a sample of the enzyme that has not been incubated at the elevated temperature. The resulting thermal denaturation profile (temperature versus residual activity) can be used to calculate the temperature at which 50 % residual activity is obtained. This value is defined as the Tm value. The Tm value is the temperature at which 50 % residual activity is obtained after 5 min incubation. The polypeptide SEQ ID NO: 3 was preincubated for 30, 60, and 300 s at pH 6.5 at 80, 85, 90, and 95 °C. Afterwards, enzyme activity was determined at standard conditions as described in example 5 and compared to the untreated polypeptide of SEQ ID NO 3, respectively. For SEQ ID NO: 3 the Tm value is between 85 °C and 90 °C (Figure 4). During pelleting for feed pellets high temperature is applied for a relatively short time period (e.g. 30 sec at about 80 °C, WO2008063309). As shown in Figure 4 the enzyme SEQ ID NO: 3 exhibits 80 % or higher enzyme activity, even when the temperature exposure is up to 60 s and up to 95 °C. Thus, the polypeptide of SEQ ID NO: 3 completely fulfills the requirements of the pelleting process.

**Example 8: Resistence against acid treatment**

To simulate conditions occurring in the gastric tract of poultry *in vitro,* the polypeptides of SEQ ID NO: 1 and SEQ ID NO: 3 were incubated at pH 3.5 at 40 °C for 30, 60 and 120 min. The remaining activity was determined by DNSA assay and compared to the activity of the untreated protein in accordance with Example 5 unless the temperature was changed to 70 °C. The parent enzyme SEQ ID NO: 1 shows a decrease in enzyme activity along with the prolonged acidic treatment. Surprisingly, this effect is diminished drastically for the enzyme SEQ ID NO: 3 lacking the carbon binding motives (Figure 5). Even at conditions occurring in the chicken intestine, more than 70 % of the initial activity can be restored after the simulated passage of the gastric tract.

**Example 9: Resistence against proteolysis**

Proteolytic stability of SEQ ID NO: 3 against pepsin was tested by incubating the enzyme for 30 and 60 minutes at 40 °C with 50 U/ml pepsin from gastric mucosa (Sigma-Aldrich) in incubation buffer (pH 3.5; 50 mM NaCl). Residual activity was determined as described in Example 5 and is shown in Figure 6. Prolonged pepsin digestion didn't affect enzymatic activity and more than 85 % residual activity could be recovered after 2 h of pepsin digestion of the enzyme.

**Example 10: Reduction of viscosity in the *in vitro* chicken intestine model**

To demonstrate experimentally the production of animal feed pellets and the gastrointestinal passage of animal feed pellets, the following procedure was chosen: Enzyme was mixed with milled feed and heated at 95 ° C for 2 minutes in a water bath. The simulation of the gastric passage was performed as described by Bedford and Classen (1993). Protease digestion was tested by incubating the feed enzyme mixture for 45 min at 40 ° C and pH 3.0 with pepsin. Passage of the feed enzyme mixture through the small intestine environment was simulated for 2 h at 40 °C and pH 6.8 and then the viscosity was determined in a spherical viscometer. The same number of units was used for SEQ ID NO: 3 and the Danisco xylanase, which can be purchased from distributors such as Biochem Zusatzstoffe Handels- und Produktionsgesellschaft mbH, was used for comparison. The SEQ ID NO: 3 achieved an equivalent result and thus shows its effectiveness in this *in vitro* assay. (see also Figure 7)

**Example 11: Viscosity reduction of wheat slurry**

In first generation ethanol production, wheat slurry is an industrial important source for sugars. At the beginning mashing is performed at high temperature (>70 °C) to avoid microbial contamination. To reduce undesirable high viscosities and enable starch accessibility xylanase and cellulase are added early in the process together with starch hydrolysing alpha amylase. To keep the slurry in a pumpable and mixable state a rapid decrease in viscosity is an urgent need and a xylanase with robust activity at high temperature is needed.

To demonstrate the ability of the polypeptides of SEQ ID NOs: 1 to 3 to reduce viscosity at high temperature, 250 g shredded wheat were mashed with 500 mL of boiling water. After gaining a homogeneous slurry different enzymes were added: 25 mg/kg Teramyl 300L, an alpha-amylase from *Bacillus licheniformis* (Sigma-Aldrich, A4862), 0.4 mg/kg SEQ ID NO: 3, 0.4 mg/kg of a hemicellulase/cellulase blend (80 % SEQ ID NO: 3, and 20 % of a modified endoglucanase from *Clostridium thermocellum* (SEQ ID NO: 11, EP17203087)) or 0.4 mg/kg Cellic CTec2 (Sigma-Aldrich, SAE0020 SIGMA). Viscosity reduction was monitored as time to complete fluidity in a stirred tank reactor equipped with one Rushton impeller (BIOSTAT Single, 2 L Univessel) at 70 °C jacket temperature and 1000 rpm. Without any enzyme no liquefaction could be observed. For calculation reason, the time to complete liquefaction using Teramyl 300L was set to 100 %. A combination of Teramyl 300L and the polypeptide of SEQ ID NO: 3 leads to a 40 % decrease in liquefaction time. A 60 % decrease could be achieved with Teramyl 300L, the polypeptide of SEQ ID NO: 3 and an additional endoglucanase from *C. thermocellum* (SEQ ID NO: 11, EP17203087), whereas the commercial available cellulose/hemicellulose mix Cellic CTec2, added in the same dosage, shows no effect (Figure 8).

**References**

Adelsberger, H., Hertel, C., Glawischnig, E., Zverlov, V.V., Schwarz, W.H., 2004. Enzyme system of Clostridium stercorarium for hydrolysis of arabinoxylan: reconstitution of the in vivo system from recombinant enzymes. Microbiology 150 (7), 2257-2266.

Bedford M. R. and Classen H. L. 1993. An In Vitro Assay for Prediction of Broiler Intestinal Viscosity and Growth When Fed Rye-Based Diets in the Presence of Exogenous Enzymes Poultry Science, Volume 72, Issue 1, 1 January 1993, Pages 137-143.

Biely, P., Vrsanská, M., Tenkanen, M., Kluepfel, D., 1997. Endo-beta-1,4-xylanase families: differences in catalytic properties. Journal of biotechnology 57 (1-3), 151-166.

Bodie, E. A., Cuevas, W. A., Koljonen, M. Five thermostable xylanases from microtetraspora flexuosa for use in delignification and/or bleaching of pulp.. WO1995029997A1, WO1995029998A1 1994

Menzen, T., Friess, W., 2013.Temperature-Ramped Studies on the Aggregation, Unfolding, and Interaction of a Therapeutic Monoclonal Antibody. Journal of Pharmaceutical Sciences , Volume 103 , Issue 2 , 445 - 455.

Bouraoui, H., Desrousseaux, M.-L., Ioannou, E., Alvira, P., Manai, M., Remond, C., Dumon, C., Fernandez-Fuentes, N., O'Donohue, M.J., 2016. The GH51 alpha-L-arabinofuranosidase from Paenibacillus sp. THS1 is multifunctional, hydrolyzing main-chain and side-chain glycosidic bonds in heteroxylans. Biotechnology for biofuels 9, 140.

Brockmeier, U., Caspers, M., Freudl, R., Jockwer A., Noll, T. and Eggert T., 2006, Systematic Screening of All Signal Peptides from Bacillus subtilis: A Powerful Strategy in Optimizing Heterologous Protein Secretion in Gram-positive Bacteria, Journal of Molecular Biology. Volume 362, Issue 3, Pages 393-402.

Carre, B., Escartin, R., Melcion, J. P., Champ, M., Roux, G., Leclercq, B. Effect of pelleting and associations with maize or wheat on the nutritive value of smooth pea (Pisum satiuum) seeds in adult cockerels. Br. Poultry Sci. 1987,28, 219-229.

Chi, WJ., Park, D.Y., Chang, YK., Hng SK. (2012). A Novel Alkaliphilic Xylanase from the Newly Isolated Mesophilic Bacillus sp. MX47: Production, Purification, and Characterization. Appl Biochem Biotechnol 168: 899.

Collins, T., Gerday, C., Feller, G., 2005. Xylanases, xylanase families and extremphilic xylanases. FEMS Microbiology Reviews, 29: 3-23.

Evans, G. I., Lewis, G. K., Ramsay, G. and Bishop, J.M., 1985. Isolation of monoclonal antibodies specific for human c-myc proto-oncogene product. Mol Cell Biol. Dec; 5 (12):3610-6.

Fukunaga, N., Iwasaki, Y., Kono, S., Kita, Y., Izumi, Y., 1998. Thermostable xylanase. US Patent 5916795. 1994.

Gems, D., Johnstone, I. L. and Clutterbuck, A. J., 1991. An autonomously replicating plasmid transforms Aspergillus nidulans at high frequency. Gene. Feb 1; 98 (1):61-7.

Guo and Sherman, 1995. Molecular Cellular Biology 15: 5983-5990.

Dutta, T., Sengupta, R., Sahoo, R., Sinha Ray, S., Bhattacharjee, A., Ghosh, S. (2007). A novel cellulase free alkaliphilic xylanase from alkali tolerant Penicillium citrinum: production, purification and characterization. Let. Appl. Microbiol. 44:206-211.

Kolenová, K., Vrš anská, M., Biely, P., 2006. Mode of end-β, 4-xylanases of families 10 and 11 on acidic xylooligosaccharides. Journal of Biotechnology, 121:338-345.

Korz, D. J., Rinas, U., Hellmuth K., Sanders, E. A., Deckwer, W.D., 1995. Simple fed-batch technique for high cell density cultivation of Escherichia coli. J. Biotechnol. 39:59-65.

Kroll, J., Steinle, A., Reichelt, R., Ewering, E., and Steinbüchel, A., 2009. Establishment of a novel anabolism based addiction system with an artificially introduced mevalonate pathway: complete stabilization of plasmids as universal application in white biotechnology. Metab Eng 11:168-177.

Laemmli, U. K., 1970. Cleavage of structural proteins during the assembly of the head of bacteriophage T4. Nature 227, 880-685.

Lombard V., Golaconda R.H., Drula E., Coutinho P.M., Henrissat B., 2014. The Carbohydrate-active enzymes database (CAZy) in 2013. Nucleic Acids Res 42:D490-D495. [PMID: 24270786].

Park, Y.S., Kai, K., Iijima, S., Kobayashi, T., 1991. Enhanced β-Galactosidase Production by High Cell-Density Culture of Recombinant Bacillus subtilis with Glucose Concentration control). Biotechnol Bioeng. 40(6), 686-696.

Prade, R. A. 1996. Xylanases: from Biology to BioTechnology, Biotechnology and Genetic Engineering Reviews, 13:1, 101-132.

Prakash, P., Jayalakshmi, S.K., Prakash, B. Rubul. M., Sreeramulu, K., 2012. Production of alkaliphilic, halotolerent, thermostable cellulase free xylanase by Bacillus halodurans PPKS-2 using agro waste: single step purification and characterization World J Microbiol Biotechnol 28: 183-192.

Niesen, F. H., Berglund, H., & Vedadi, M. (2007). The use of differential scanning fluorimetry to detect ligand interactions that promote protein stability. Nature protocols, 2(9), 2212-2221

Romanos, M. A., Scorer, C. A. and Jeffrey, J. C., 1992. Foreign Gene Expression in Yeast: a Review. YEAST VOL. 8: 423-488.

Sambrook, J, Russell, D. W., 2001.Molecular cloning: a laboratory manual 3rd edition. Coldspring-Harbour Laboratory Press, UK.

Sambrook, J., Fritsch, E. F., Maniatis. T., 1989. Molecular cloning: a laboratory manual, 2nd ed. Cold Spring Harbor Laboratory Press, Cold Spring Harbor, NY.

Simonen, M. and Palva, I., 1993. Protein secretion in Bacillus species. Microbiological Reviews 57: 109-137.

Schwarz, W. H., Adelsberger, H., Jauris, S., Hertel, C., Funk, B., Staudenbauer, W.L. 1990. "Xylan degradation by the thermophile Clostridium stercorarium: cloning and expression of xylanase, beta-D-xylosidase, and alpha-L-arabinofuranosidase genes in Escherichia coli." Biochem Biophys Res Commun 170(1): 368-374.

Weber, K., Osborn, M., 1969.The reliability of molecular weight determinations by dodecyl sulfate-polyacrylamide gel electrophoresis. J Biol Chem. 1969 Aug 25;244(16):4406-12.

Wilson, I. A., Niman, H. L., Houghten, R. A., Cherenson, A. R., Conolly, M. L., Lerner, R. A., 1984. The structure of an antigenic determinant in a protein. Cell. ; 37(3):767-78.

Wood TM, Bhat KM. Methods for measuring cellulase activities. Methods in enzymology, Vol. 160. 1988. p. 87 - 112.

## Claims

1. A polypeptide, comprising, essentially consisting of or consisting of a polypeptide which has at least 75 % amino acid sequence identity to the polypeptide according to SEQ ID NOs: 2 or 3, preferably of SEQ ID NO: 3, wherein said polypeptide has xylanase activity, and with the proviso that the polypeptide is not the polypeptide of SEQ ID NO: 1.

2. The polypeptide according to claim 1, wherein said polypeptide shows improved thermostability and/or resistance against acid treatment and/or an increased enzyme activity, wherein

   - improved thermostability means that said polypeptide displays a $Tm_{on}$ of 70 °C or higher and/or displays a $Tm_{50}$ of 80 °C or higher and/or displays a Tm in the range of 80 °C to 90 °C;

- improved resistance against acid treatment means that an enzyme activity of at least 70 % is retained after treatment at low pH, such as a pH at 2.5 to 5.5; and
- increased enzyme activity means that the specific enzyme activity is increased at least 1.6 fold compared to the wildtype enzyme.

**3.** The polypeptide according to any one of the preceding claims, wherein said polypeptide comprises, essentially consists of or consists of a polypeptide having at least 75 % amino acid sequence identity to a polypeptide of SEQ ID NO: 3, and wherein said polypeptide of SEQ ID NO: 3 shows a 1.6 fold increase of the specific enzyme activity and a $Tm_{on}$ of 81.2 °C and/or a $Tm_{50}$ of 85.7 °C.

**4.** The polypeptide according to any one of claims 1 to 3, wherein said polypeptide displays at least 40 % enzyme activity, in particular xylanase activity, in a pH range from 5.5 to 9.5, and/or in a temperature range from 37 °C to 80 °C or higher.

**5.** The polypeptide according to any one of claims 1 to 4, wherein the enzyme activity is retained after acidic treatment of the polypeptide in the pH range from 2.0 to 5.5.

**6.** A nucleic acid molecule comprising, consisting essentially of or consisting of a nucleic acid sequence of SEQ ID NO: 9 or 10 encoding the polypeptide according to SEQ ID NO: 2 or 3.

**7.** An expression vector comprising the nucleic acid molecule as claimed in claim 6.

**8.** A host cell comprising the nucleic acid sequence of SEQ ID NO: 9 or 10 as claimed in claim 6 or the expression vector as claimed in claim 7, wherein said host cell expresses the polypeptide according to SEQ ID NO: 2 or 3.

**9.** A method for producing the polypeptide of SEQ ID NOs: 2 or 3, preferably of SEQ ID NO: 3, the method comprising culturing a host cell as claimed in claim 8 under conditions permitting the production of the polypeptide, and recovering the polypeptide from the culture.

**10.** A composition for addition to biomass or hemicellulose containing material, said composition comprising a polypeptide which has at least 75 % amino acid sequence identity to the polypeptide according to SEQ ID NO 1, 2 or 3, and optionally at least one formulating agent, excipient, stabilizer and/or a preservative.

**11.** The composition according to claim 10, wherein said composition is a liquid formulation, such as a solution or suspension, or a dry formulation, such as a powder or granulate.

**12.** The composition according to claim 10 or 11, wherein said composition comprises a sugar as heat stabilizing agent, which is e.g. selected from sucrose, trehalose, sorbose, melezitose, verbascose, melibiose, sucralose and raffinose, or, when said composition is a liquid formulation, comprises a solvent, such as glycerol or water.

**13.** The polypeptide or the composition according to any one of the preceding claims, wherein said polypeptide modifies the content of hemicellulose components, in particular the xylan content, to loosen up e.g. compact structure or to reduce high viscosity of biomass or hemicellulose containing material.

**14.** Use of the polypeptide or the composition according to any one of the preceding claims during the production of animal feed, pulp and paper, bioenergy and in brewery or malting.

**Amended claims in accordance with Rule 137(2) EPC.**

**1.** A polypeptide, comprising or consisting of a polypeptide which has at least 75 % amino acid sequence identity to the polypeptide according to SEQ ID NOs: 2 or 3, preferably of SEQ ID NO: 3, wherein said polypeptide has xylanase activity, and with the proviso that the polypeptide which has at least 75 % amino acid sequence identity to the polypeptide of SEQ ID NOs: 2 or 3 is not the polypeptide of SEQ ID NO: 1; **characterized in that** said polypeptide shows improved thermostability and/or resistance against acid treatment and/or an increased enzyme activity compared to the polypeptide of SEQ ID NO: 1, wherein

- improved thermostability means that said polypeptide displays a $Tm_{on}$ of 70 °C or higher and/or displays a

$Tm_{50}$ of 80 °C or higher and/or displays a Tm in the range of 80 °C to 90 °C;
- improved resistance against acid treatment means that an enzyme activity of at least 70 % is retained after treatment at low pH, such as a pH at 2.5 to 5.5; and
- increased enzyme activity means that the specific enzyme activity is increased at least 1.6 fold.

**2.** The polypeptide according to claim 1, wherein said polypeptide comprises or consists of a polypeptide having at least 75 % amino acid sequence identity to a polypeptide of SEQ ID NO: 3, and wherein said polypeptide of SEQ ID NO: 3 shows a 1.6 fold increase of the specific enzyme activity and a $Tm_{on}$ of 81.2 °C and/or a $Tm_{50}$ of 85.7 °C.

**3.** The polypeptide according to claim 1 or 2, wherein said polypeptide displays at least 40 % enzyme activity, in particular xylanase activity, over a pH range from 5.5 to 9.5, and/or over a temperature range from 37 °C to 80 °C.

**4.** The polypeptide according to any one of claims 1 to 3, wherein the enzyme activity is retained after acidic treatment of the polypeptide in the pH range from 2.0 to 5.5.

**5.** A nucleic acid molecule consisting of a nucleic acid sequence of SEQ ID NO: 9 encoding the polypeptide according to SEQ ID NO: 3.

**6.** An expression vector comprising the nucleic acid molecule as claimed in claim 5.

**7.** A host cell comprising the nucleic acid sequence of SEQ ID NO: 9 as claimed in claim 5 or the expression vector as claimed in claim 6, wherein said host cell expresses the polypeptide according to SEQ ID NO: 3.

**8.** A method for producing the polypeptide of SEQ ID NO: 3, the method comprising culturing a host cell as claimed in claim 7 under conditions permitting the production of the polypeptide, and recovering the polypeptide from the culture.

**9.** A composition for addition to biomass or hemicellulose containing material, said composition comprising a polypeptide as claimed in claim 1, and optionally at least one formulating agent, excipient, stabilizer and/or a preservative.

**10.** The composition according to claim 9, wherein said composition is a liquid formulation, such as a solution or suspension, or a dry formulation, such as a powder or granulate.

**11.** The composition according to claim 9 or 10, wherein said composition comprises a sugar as heat stabilizing agent, which is e.g. selected from sucrose, trehalose, sorbose, melezitose, verbascose, melibiose, sucralose and raffinose, or, when said composition is a liquid formulation, comprises a solvent, such as glycerol or water.

**12.** The polypeptide or the composition according to any one of the preceding claims, wherein said polypeptide modifies the content of hemicellulose components, in particular the xylan content, to loosen up e.g. compact structure or to reduce high viscosity of biomass or hemicellulose containing material.

**13.** Use of the polypeptide or the composition according to any one of the preceding claims during the production of animal feed, pulp and paper, bioenergy and in brewery or malting.

**Figure 1**

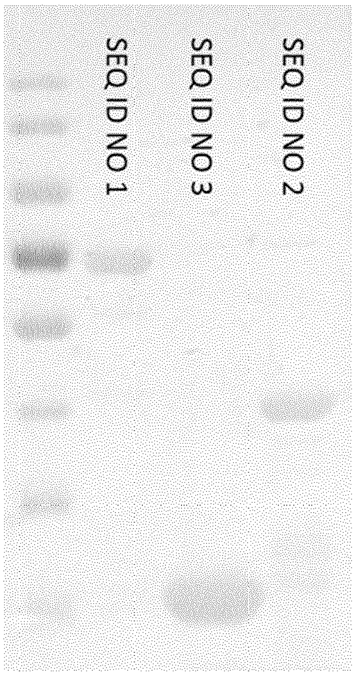


**Figure 2**

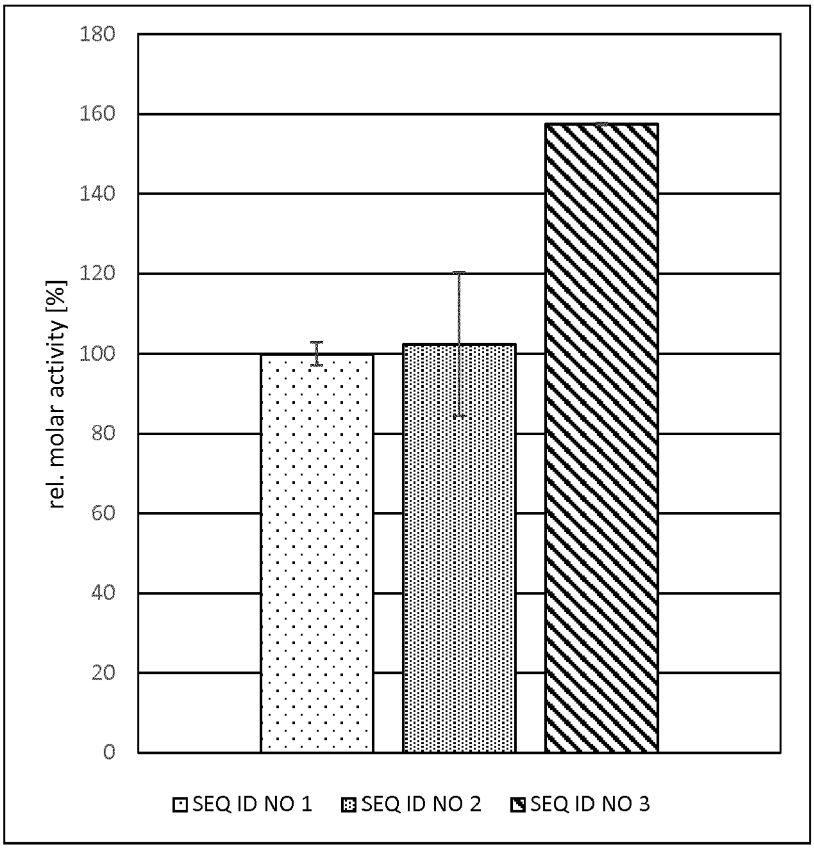

**Figure 3**

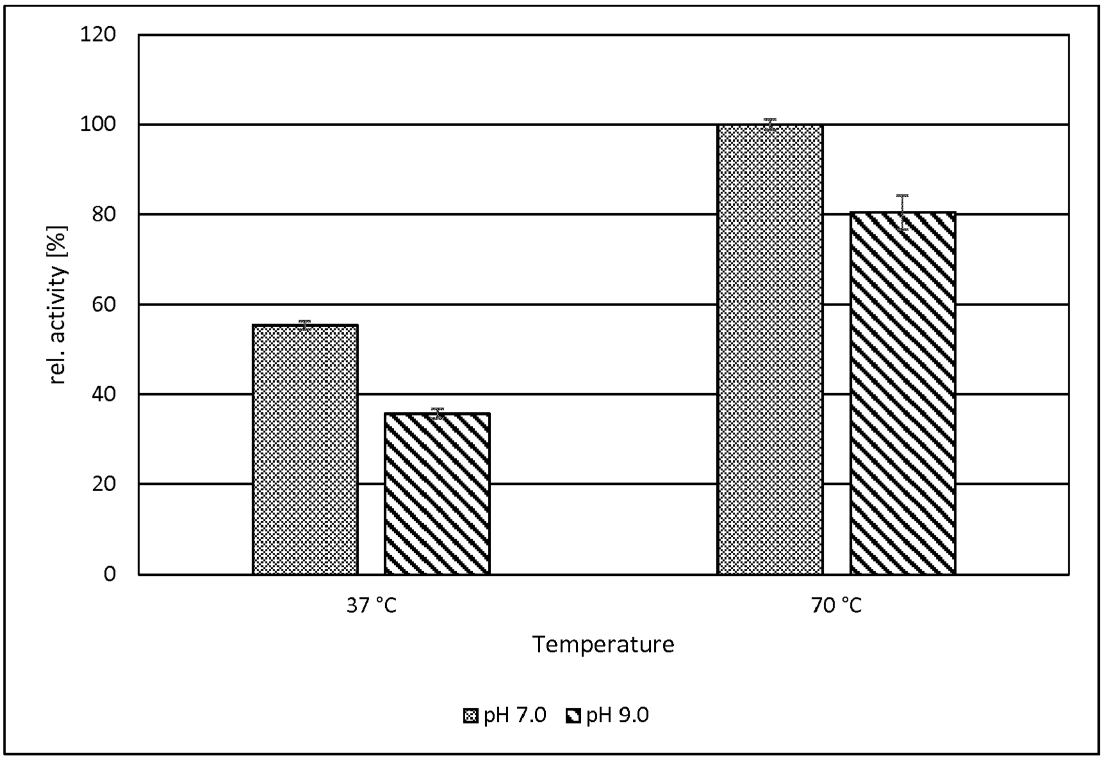


**Figure 4**

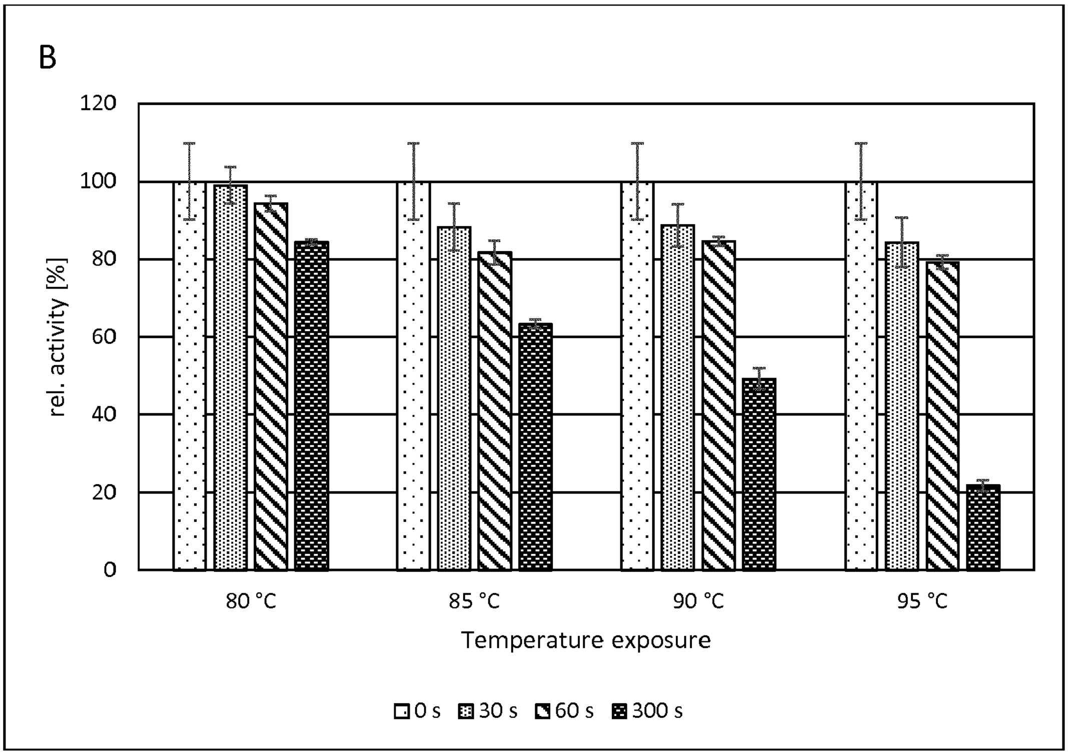

Figure 5

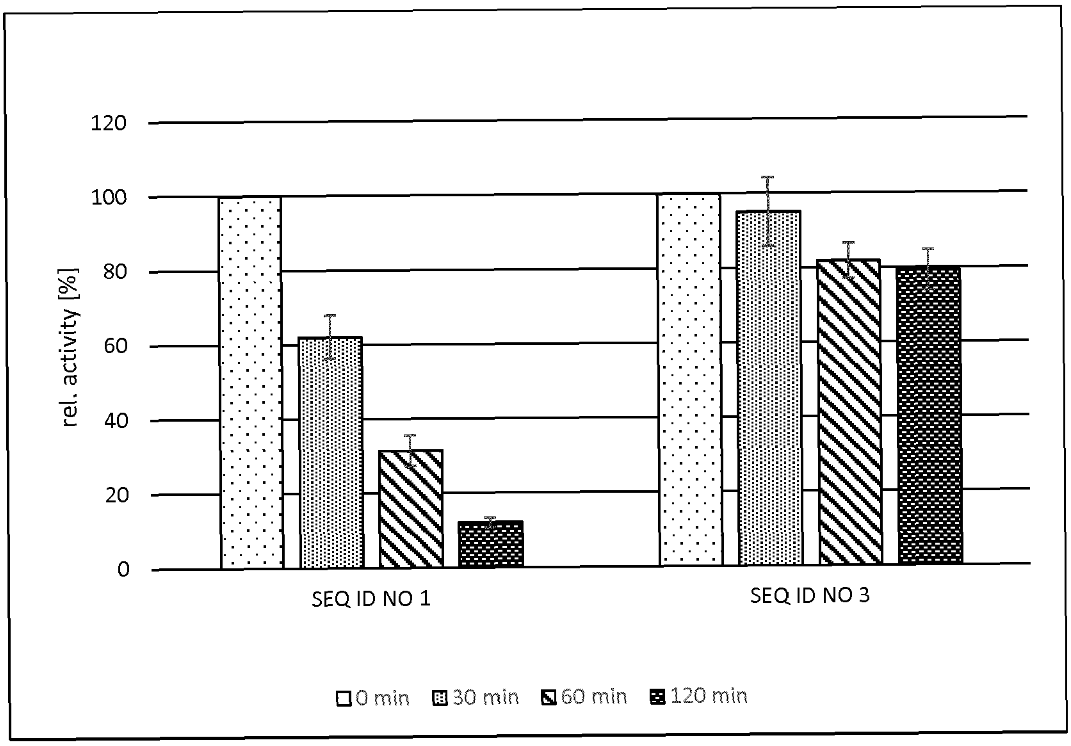


Figure 6

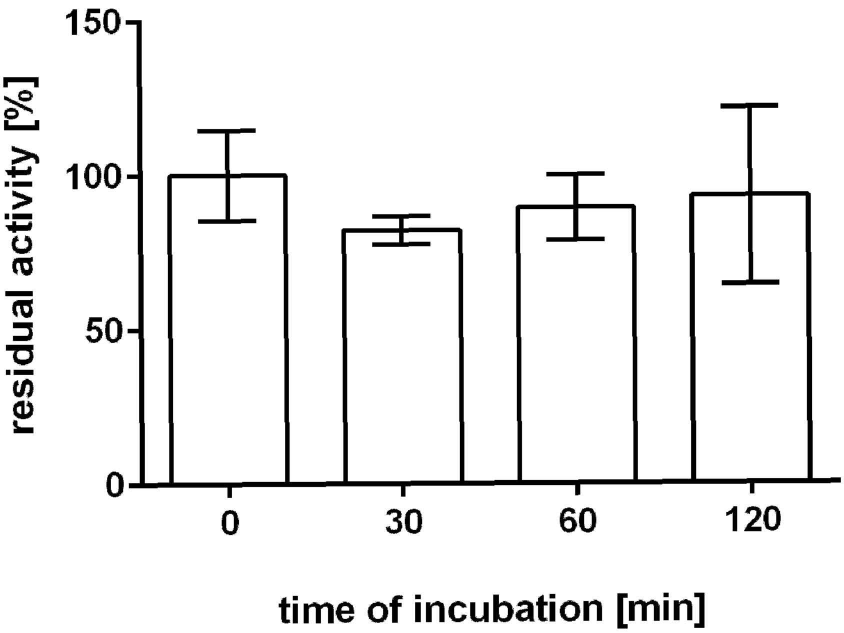

Figure 7

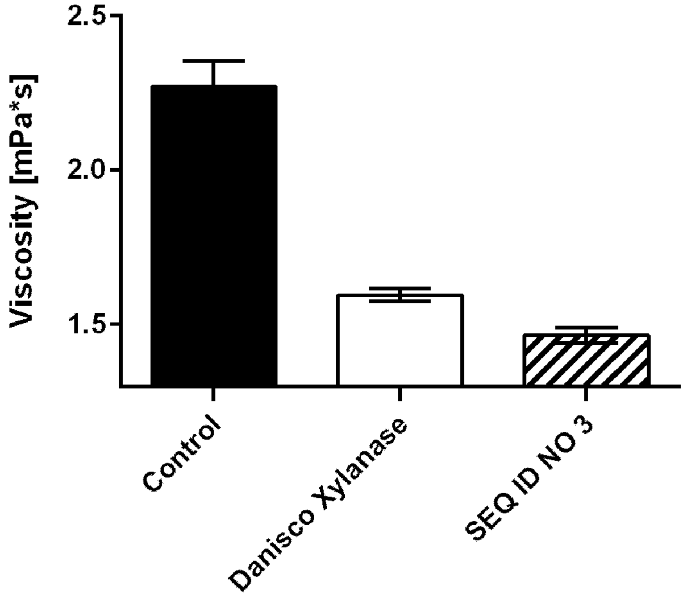


Figure 8

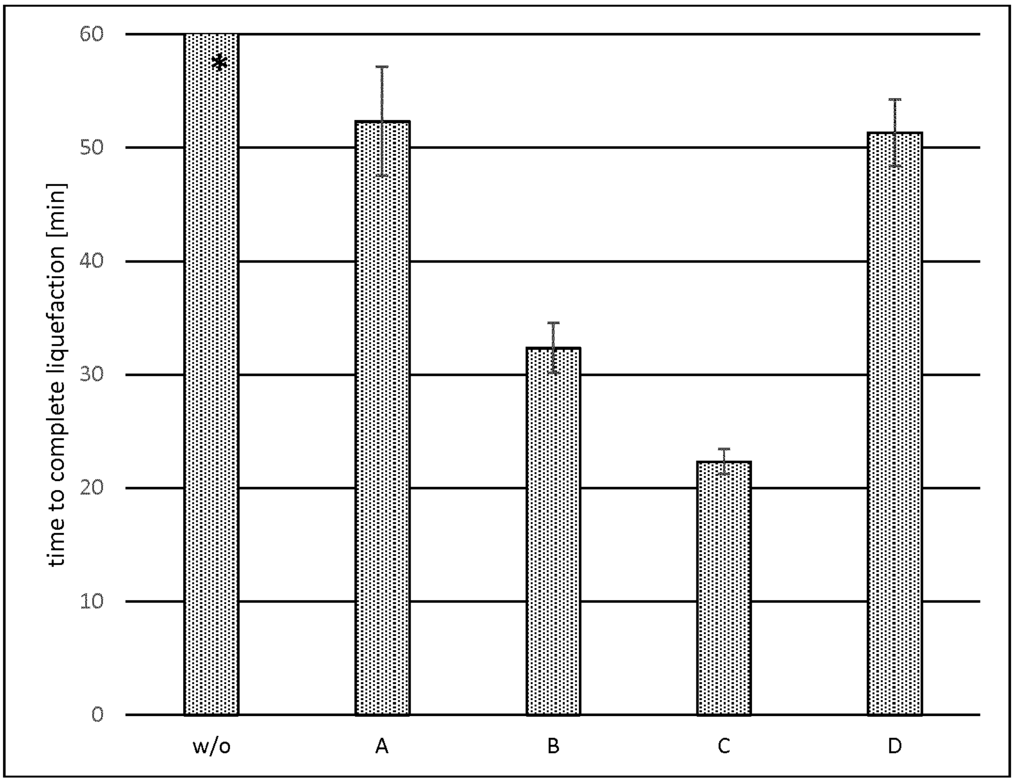

Europäisches Patentamt
European Patent Office
Office européen des brevets

# EUROPEAN SEARCH REPORT

Application Number
EP 17 20 8848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 22 November 2017 (2017-11-22), "RecName: Full=Endo-1,4-beta-xylanase {ECO:0000256¦PROSITE-ProRule:PRU01097}; EC=3.2.1.8 {ECO:0000256¦PROSITE-ProRule:PRU01097};", XP002780481, retrieved from EBI accession no. UNIPROT:A0A1B1YEM2 Database accession no. A0A1B1YEM2 | 1,6-8 | INV. C12N9/24 D21C5/00 D21H17/00 A23K10/14 |
| Y | * the whole document * | 4,5,9-14 | |
| X | WO 2007/115407 A1 (CA NAT RESEARCH COUNCIL [CA]; SUNG WING L [CA]) 18 October 2007 (2007-10-18) | 1,6,8 | |
| Y | * abstract * * page 12, line 17 - line 24; claims 1-39; figure 1 * * paragraph [0002] - paragraph [0006] * | 4,5,9-14 | |
| Y | NG CHOONG HEY ET AL: "Purification and Characterization of a GH11 Xylanase from Biobutanol-ProducingClostridium beijerinckiiG117", APPLIED BIOCHEMISTRY AND BIOTECHNOLOGY ; PART A: ENZYME ENGINEERING AND BIOTECHNOLOGY, HUMANA PRESS INC, NEW YORK, vol. 175, no. 6, 7 January 2015 (2015-01-07), pages 2832-2844, XP035460893, ISSN: 0273-2289, DOI: 10.1007/S12010-014-1470-5 [retrieved on 2015-01-07] * abstract; figures 4-6 * * page 2840, paragraph 4 - page 2841, paragraph 1 * | 4,5,9-14 | TECHNICAL FIELDS SEARCHED (IPC) C12N D21C D21H A23K |

-/--

The present search report has been drawn up for all claims

1

| Place of search | Date of completion of the search | Examiner |
|---|---|---|
| The Hague | 24 April 2018 | Gurdjian, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 8848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | SAKKA K ET AL: "NUCLEOTIDE SEQUENCE OF THE CLOSTRIDIUM STERCORARIUM XYNA GENE ENCODING XYLANASE A: IDENTIFICATION OF CATALYTIC AND CELLULOSE BINDING DOMAINS", BIOSCIENCE BIOTECHNOLOGY BIOCHEMIS, JAPAN SOCIETY FOR BIOSCIENCE, BIOTECHNOLOGY, AND AGROCHEMISTRY, TOKYO, JAPAN, vol. 57, no. 2, 1 January 1993 (1993-01-01), pages 273-277, XP009013367, ISSN: 0916-8451 * figures 1-4 * ----- | 1-14 | |
| A | SHUNSUKE ICHIKAWA ET AL: "Carbohydrate-binding modules influence substrate specificity of an endoglucanase from Clostridium thermocellum", BIOSCIENCE BIOTECHNOLOGY BIOCHEMISTRY., vol. 80, no. 1, 30 July 2015 (2015-07-30), pages 188-192, XP055448779, TOKYO, JAPAN ISSN: 0916-8451, DOI: 10.1080/09168451.2015.1069696 * abstract; figure 1 * ----- | 1-14 | TECHNICAL FIELDS SEARCHED (IPC) |

The present search report has been drawn up for all claims

| Place of search | Date of completion of the search | Examiner |
|---|---|---|
| The Hague | 24 April 2018 | Gurdjian, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

page 2 of 2

## ANNEX TO THE EUROPEAN SEARCH REPORT ON EUROPEAN PATENT APPLICATION NO. EP 17 20 8848

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2018

| Patent document cited in search report | Publication date | Patent family member(s) | Publication date |
|---|---|---|---|
| WO 2007115407 A1 | 18-10-2007 | AR 060433 A1 | 18-06-2008 |
| | | BR PI0711378 A2 | 22-11-2011 |
| | | CA 2649852 A1 | 18-10-2007 |
| | | CN 101460615 A | 17-06-2009 |
| | | EP 2002000 A1 | 17-12-2008 |
| | | US 2009148923 A1 | 11-06-2009 |
| | | WO 2007115391 A1 | 18-10-2007 |
| | | WO 2007115407 A1 | 18-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008063309 A
- WO 0056900 A
- WO 9600787 A
- WO 9533836 A
- WO 0024883 A
- EP 17203087 A
- WO 1995029997 A1
- WO 1995029998 A1
- US 5916795 A


### Non-patent literature cited in the description


- Useful proteins from recombinant bacteria. Scientific American, 1980, vol. 242, 74-94
- Protein Purification. VCH Publishers, 1989
- **ADELSBERGER, H. ; HERTEL, C. ; GLAWISCHNIG, E. ; ZVERLOV, V.V. ; SCHWARZ, W.H.** Enzyme system of Clostridium stercorarium for hydrolysis of arabinoxylan: reconstitution of the in vivo system from recombinant enzymes. *Microbiology,* 2004, vol. 150 (7), 2257-2266
- **BEDFORD M. R. ; CLASSEN H. L.** *An In Vitro Assay for Prediction of Broiler Intestinal Viscosity and Growth When Fed Rye-Based Diets in the Presence of Exogenous Enzymes Poultry Science,* 01 January 1993, vol. 72 (1), 137-143
- **BIELY, P. ; VRSANSKÁ, M. ; TENKANEN, M. ; KLUEPFEL, D.** Endo-beta-1,4-xylanase families: differences in catalytic properties. *Journal of biotechnology,* 1997, vol. 57 (1-3), 151-166
- **MENZEN, T. ; FRIESS, W.** Temperature-Ramped Studies on the Aggregation, Unfolding, and Interaction of a Therapeutic Monoclonal Antibody. *Journal of Pharmaceutical Sciences,* 2013, vol. 103 (2), 445-455
- **BOURAOUI, H. ; DESROUSSEAUX, M.-L. ; IOANNOU, E. ; ALVIRA, P. ; MANAI, M. ; REMOND, C. ; DUMON, C. ; FERNANDEZ-FUENTES, N. ; O'DONOHUE, M.J.** The GH51 alpha-L-arabinofuranosidase from Paenibacillus sp. THS1 is multifunctional, hydrolyzing main-chain and side-chain glycosidic bonds in heteroxylans. *Biotechnology for biofuels,* 2016, vol. 9, 140
- **BROCKMEIER, U. ; CASPERS, M. ; FREUDL, R. ; JOCKWER A. ; NOLL, T. ; EGGERT T.** Systematic Screening of All Signal Peptides from Bacillus subtilis: A Powerful Strategy in Optimizing Heterologous Protein Secretion in Gram-positive Bacteria. *Journal of Molecular Biology,* 2006, vol. 362 (3), 393-402
- **CARRE, B. ; ESCARTIN, R. ; MELCION, J. P. ; CHAMP, M. ; ROUX, G. ; LECLERCQ, B.** Effect of pelleting and associations with maize or wheat on the nutritive value of smooth pea (Pisum satiuum) seeds in adult cockerels. *Br. Poultry Sci.,* 1987, vol. 28, 219-229
- **CHI, WJ. ; PARK, D.Y. ; CHANG, YK. ; HNG SK.** A Novel Alkaliphilic Xylanase from the Newly Isolated Mesophilic Bacillus sp. MX47: Production, Purification, and Characterization. *Appl Biochem Biotechnol,* 2012, vol. 168, 899
- **COLLINS, T. ; GERDAY, C. ; FELLER, G.** Xylanases, xylanase families and extremphilic xylanases. *FEMS Microbiology Reviews,* 2005, vol. 29, 3-23
- **EVANS, G. I. ; LEWIS, G. K. ; RAMSAY, G. ; BISHOP, J.M.** Isolation of monoclonal antibodies specific for human c-myc proto-oncogene product. *Mol Cell Biol.,* December 1985, vol. 5 (12), 3610-6
- **GEMS, D. ; JOHNSTONE, I. L. ; CLUTTERBUCK, A. J.** An autonomously replicating plasmid transforms Aspergillus nidulans at high frequency. *Gene,* 01 February 1991, vol. 98 (1), 61-7
- **GUO ; SHERMAN.** *Molecular Cellular Biology,* 1995, vol. 15, 5983-5990
- **DUTTA, T. ; SENGUPTA, R. ; SAHOO, R. ; SINHA RAY, S. ; BHATTACHARJEE, A. ; GHOSH, S.** A novel cellulase free alkaliphilic xylanase from alkali tolerant Penicillium citrinum: production, purification and characterization. *Let. Appl. Microbiol.,* 2007, vol. 44, 206-211
- **KORZ, D. J. ; RINAS, U. ; HELLMUTH K. ; SANDERS, E. A. ; DECKWER, W.D.** Simple fed-batch technique for high cell density cultivation of Escherichia coli. *J. Biotechnol.,* 1995, vol. 39, 59-65

- **KROLL, J. ; STEINLE, A. ; REICHELT, R. ; EWERING, E. ; STEINBÜCHEL, A.** Establishment of a novel anabolism based addiction system with an artificially introduced mevalonate pathway: complete stabilization of plasmids as universal application in white biotechnology. *Metab Eng,* 2009, vol. 11, 168-177
- **LAEMMLI, U. K.** Cleavage of structural proteins during the assembly of the head of bacteriophage T4. *Nature,* 1970, vol. 227, 880-685
- **LOMBARD V. ; GOLACONDA R.H. ; DRULA E. ; COUTINHO P.M. ; HENRISSAT B.** The Carbohydrate-active enzymes database (CAZy). *Nucleic Acids Res,* 2013, vol. 42, D490-D495
- **PARK, Y.S. ; KAI, K. ; LIJIMA, S. ; KOBAYASHI, T.** Enhanced β-Galactosidase Production by High Cell-Density Culture of Recombinant Bacillus subtilis with Glucose Concentration control. *Biotechnol Bioeng.,* 1991, vol. 40 (6), 686-696
- **PRADE, R. A.** *Xylanases: from Biology to BioTechnology, Biotechnology and Genetic Engineering Reviews,* 1996, vol. 13 (1), 101-132
- **PRAKASH, P. ; JAYALAKSHMI, S.K. ; PRAKASH, B. ; RUBUL. M. ; SREERAMULU, K.** Production of alkaliphilic, halotolerent, thermostable cellulase free xylanase by Bacillus halodurans PPKS-2 using agro waste: single step purification and characterization. *World J Microbiol Biotechnol,* 2012, vol. 28, 183-192
- **NIESEN, F. H. ; BERGLUND, H. ; VEDADI, M.** The use of differential scanning fluorimetry to detect ligand interactions that promote protein stability. *Nature protocols,* 2007, vol. 2 (9), 2212-2221
- **ROMANOS, M. A. ; SCORER, C. A. ; JEFFREY, J. C.** Foreign Gene Expression in Yeast: a Review. *YEAST,* 1992, vol. 8, 423-488
- **SAMBROOK, J ; RUSSELL, D. W.** Molecular cloning: a laboratory manual. Coldspring-Harbour Laboratory Press, 2001
- **SAMBROOK, J. ; FRITSCH, E. F. ; MANIATIS. T.** Molecular cloning: a laboratory manual. Cold Spring Harbor Laboratory Press, 1989
- **SIMONEN, M. ; PALVA, I.** *Protein secretion in Bacillus species. Microbiological Reviews,* 1993, vol. 57, 109-137
- **SCHWARZ, W. H. ; ADELSBERGER, H. ; JAURIS, S. ; HERTEL, C. ; FUNK, B. ; STAUDENBAUER, W.L.** Xylan degradation by the thermophile Clostridium stercorarium: cloning and expression of xylanase, beta-D-xylosidase, and alpha-L-arabinofuranosidase genes in Escherichia coli. *Biochem Biophys Res Commun,* 1990, vol. 170 (1), 368-374
- **WEBER, K. ; OSBORN, M.** The reliability of molecular weight determinations by dodecyl sulfate-polyacrylamide gel electrophoresis. *J Biol Chem.,* 25 August 1969, vol. 244 (16), 4406-12
- **WILSON, I. A. ; NIMAN, H. L. ; HOUGHTEN, R. A. ; CHERENSON, A. R. ; CONOLLY, M. L. ; LERNER, R. A.** The structure of an antigenic determinant in a protein. *Cell,* 1984, vol. 37 (3), 767-78
- **WOOD TM ; BHAT KM.** Methods for measuring cellulase activities. *Methods in enzymology,* 1988, vol. 160, 87-112

SEQUENCE LISTING

```
<110>  Technische Universität München

<120>  New Xylanase with improved thermostability and increased enzyme
       activity on arabinoxylan

<130>  MTU108EP

<160>  12

<170>  PatentIn version 3.5

<210>  1
<211>  621
<212>  PRT
<213>  Clostridium stercorarium

<400>  1

Gly Arg Ile Ile Tyr Asp Asn Glu Thr Gly Thr His Gly Gly Tyr Asp
1               5                   10                  15


Tyr Glu Leu Trp Lys Asp Tyr Gly Asn Thr Ile Met Glu Leu Asn Asp
            20                  25                  30


Gly Gly Thr Phe Ser Cys Gln Trp Ser Asn Ile Gly Asn Ala Leu Phe
        35                  40                  45


Arg Lys Gly Arg Lys Phe Asn Ser Asp Lys Thr Tyr Gln Glu Leu Gly
    50                  55                  60


Asp Ile Val Val Glu Tyr Gly Cys Asp Tyr Asn Pro Asn Gly Asn Ser
65                  70                  75                  80


Tyr Leu Cys Val Tyr Gly Trp Thr Arg Asn Pro Leu Val Glu Tyr Tyr
                85                  90                  95


Ile Val Glu Ser Trp Gly Ser Trp Arg Pro Pro Gly Ala Thr Pro Lys
            100                 105                 110


Gly Thr Ile Thr Val Asp Gly Gly Thr Tyr Glu Ile Tyr Glu Thr Thr
        115                 120                 125


Arg Val Asn Gln Pro Ser Ile Asp Gly Thr Ala Thr Phe Gln Gln Tyr
    130                 135                 140


Trp Ser Val Arg Thr Ser Lys Arg Thr Ser Gly Thr Ile Ser Val Thr
145                 150                 155                 160


Glu His Phe Lys Gln Trp Glu Arg Met Gly Met Arg Met Gly Lys Met
                165                 170                 175
```

```
Tyr Glu Val Ala Leu Thr Val Glu Gly Tyr Gln Ser Ser Gly Tyr Ala
            180                 185                 190

Asn Val Tyr Lys Asn Glu Ile Arg Ile Gly Ala Asn Pro Thr Pro Ala
        195                 200                 205

Pro Ser Gln Ser Pro Ile Arg Arg Asp Ala Phe Ser Ile Ile Glu Ala
    210                 215                 220

Glu Glu Tyr Asn Ser Thr Asn Ser Ser Thr Leu Gln Val Ile Gly Thr
225                 230                 235                 240

Pro Asn Asn Gly Arg Gly Ile Gly Tyr Ile Glu Asn Gly Asn Thr Val
                245                 250                 255

Thr Tyr Ser Asn Ile Asp Phe Gly Ser Gly Ala Thr Gly Phe Ser Ala
            260                 265                 270

Thr Val Ala Thr Glu Val Asn Thr Ser Ile Gln Ile Arg Ser Asp Ser
        275                 280                 285

Pro Thr Gly Thr Leu Leu Gly Thr Leu Tyr Val Ser Ser Thr Gly Ser
    290                 295                 300

Trp Asn Thr Tyr Gln Thr Val Ser Thr Asn Ile Ser Lys Ile Thr Gly
305                 310                 315                 320

Val His Asp Ile Val Leu Val Phe Ser Gly Pro Val Asn Val Asp Asn
                325                 330                 335

Phe Ile Phe Ser Arg Ser Ser Pro Val Pro Ala Pro Gly Asp Asn Thr
            340                 345                 350

Arg Asp Ala Tyr Ser Ile Ile Gln Ala Glu Asp Tyr Asp Ser Ser Tyr
        355                 360                 365

Gly Pro Asn Leu Gln Ile Phe Ser Leu Pro Gly Gly Gly Ser Ala Ile
    370                 375                 380

Gly Tyr Ile Glu Asn Gly Tyr Ser Thr Thr Tyr Asn Asn Val Asn Phe
385                 390                 395                 400

Ala Asn Gly Leu Ser Ser Ile Thr Ala Arg Val Ala Thr Gln Ile Ser
                405                 410                 415

Thr Ser Ile Gln Val Arg Ala Gly Gly Ala Thr Gly Thr Leu Leu Gly
            420                 425                 430
```

Thr Ile Tyr Val Pro Ser Thr Asn Ser Trp Asp Ser Tyr Gln Asn Val
435 440 445

Thr Ala Asn Leu Ser Asn Ile Thr Gly Val His Asp Ile Thr Leu Val
450 455 460

Phe Ser Gly Pro Val Asn Val Asp Tyr Phe Val Phe Thr Pro Ala Asn
465 470 475 480

Val Asn Ser Gly Pro Thr Ser Pro Val Gly Gly Thr Arg Ser Ala Phe
485 490 495

Ser Asn Ile Gln Ala Glu Asp Tyr Asp Ser Ser Tyr Gly Pro Asn Leu
500 505 510

Gln Ile Phe Ser Leu Pro Gly Gly Gly Ser Ala Ile Gly Tyr Ile Glu
515 520 525

Asn Gly Tyr Ser Thr Thr Tyr Lys Asn Ile Asp Phe Gly Asp Gly Ala
530 535 540

Thr Ser Val Thr Ala Arg Val Ala Thr Gln Asn Ala Thr Thr Ile Gln
545 550 555 560

Val Arg Leu Gly Ser Pro Ser Gly Thr Leu Leu Gly Thr Ile Tyr Val
565 570 575

Gly Ser Thr Gly Ser Phe Asp Thr Tyr Arg Asp Val Ser Ala Thr Ile
580 585 590

Ser Asn Thr Ala Gly Val Lys Asp Ile Val Leu Val Phe Ser Gly Pro
595 600 605

Val Asn Val Asp Trp Phe Val Phe Ser Lys Ser Gly Thr
610 615 620

<210> 2
<211> 343
<212> PRT
<213> Artificial sequence

<220>
<223> Synthetic or recombinant polypeptide

<400> 2

Gly Arg Ile Ile Tyr Asp Asn Glu Thr Gly Thr His Gly Gly Tyr Asp
1 5 10 15

```
Tyr Glu Leu Trp Lys Asp Tyr Gly Asn Thr Ile Met Glu Leu Asn Asp
            20                  25                  30

Gly Gly Thr Phe Ser Cys Gln Trp Ser Asn Ile Gly Asn Ala Leu Phe
        35                  40                  45

Arg Lys Gly Arg Lys Phe Asn Ser Asp Lys Thr Tyr Gln Glu Leu Gly
    50                  55                  60

Asp Ile Val Val Glu Tyr Gly Cys Asp Tyr Asn Pro Asn Gly Asn Ser
65                  70                  75                  80

Tyr Leu Cys Val Tyr Gly Trp Thr Arg Asn Pro Leu Val Glu Tyr Tyr
                85                  90                  95

Ile Val Glu Ser Trp Gly Ser Trp Arg Pro Pro Gly Ala Thr Pro Lys
            100                 105                 110

Gly Thr Ile Thr Val Asp Gly Gly Thr Tyr Glu Ile Tyr Glu Thr Thr
        115                 120                 125

Arg Val Asn Gln Pro Ser Ile Asp Gly Thr Ala Thr Phe Gln Gln Tyr
    130                 135                 140

Trp Ser Val Arg Thr Ser Lys Arg Thr Ser Gly Thr Ile Ser Val Thr
145                 150                 155                 160

Glu His Phe Lys Gln Trp Glu Arg Met Gly Met Arg Met Gly Lys Met
                165                 170                 175

Tyr Glu Val Ala Leu Thr Val Glu Gly Tyr Gln Ser Ser Gly Tyr Ala
            180                 185                 190

Asn Val Tyr Lys Asn Glu Ile Arg Ile Gly Ala Asn Pro Thr Pro Ala
        195                 200                 205

Pro Ser Gln Ser Pro Ile Arg Arg Asp Ala Phe Ser Ile Ile Glu Ala
    210                 215                 220

Glu Glu Tyr Asn Ser Thr Asn Ser Ser Thr Leu Gln Val Ile Gly Thr
225                 230                 235                 240

Pro Asn Asn Gly Arg Gly Ile Gly Tyr Ile Glu Asn Gly Asn Thr Val
                245                 250                 255

Thr Tyr Ser Asn Ile Asp Phe Gly Ser Gly Ala Thr Gly Phe Ser Ala
            260                 265                 270
```

Thr Val Ala Thr Glu Val Asn Thr Ser Ile Gln Ile Arg Ser Asp Ser
275 280 285

Pro Thr Gly Thr Leu Leu Gly Thr Leu Tyr Val Ser Ser Thr Gly Ser
290 295 300

Trp Asn Thr Tyr Gln Thr Val Ser Thr Asn Ile Ser Lys Ile Thr Gly
305 310 315 320

Val His Asp Ile Val Leu Val Phe Ser Gly Pro Val Asn Val Asp Asn
325 330 335

Phe Ile Phe Ser Arg Ser Ser
340

<210> 3
<211> 204
<212> PRT
<213> Artificial sequence

<220>
<223> Synthetic or recombinant polypeptide

<400> 3

Gly Arg Ile Ile Tyr Asp Asn Glu Thr Gly Thr His Gly Gly Tyr Asp
1 5 10 15

Tyr Glu Leu Trp Lys Asp Tyr Gly Asn Thr Ile Met Glu Leu Asn Asp
20 25 30

Gly Gly Thr Phe Ser Cys Gln Trp Ser Asn Ile Gly Asn Ala Leu Phe
35 40 45

Arg Lys Gly Arg Lys Phe Asn Ser Asp Lys Thr Tyr Gln Glu Leu Gly
50 55 60

Asp Ile Val Val Glu Tyr Gly Cys Asp Tyr Asn Pro Asn Gly Asn Ser
65 70 75 80

Tyr Leu Cys Val Tyr Gly Trp Thr Arg Asn Pro Leu Val Glu Tyr Tyr
85 90 95

Ile Val Glu Ser Trp Gly Ser Trp Arg Pro Pro Gly Ala Thr Pro Lys
100 105 110

Gly Thr Ile Thr Val Asp Gly Gly Thr Tyr Glu Ile Tyr Glu Thr Thr
115 120 125

```
Arg Val Asn Gln Pro Ser Ile Asp Gly Thr Ala Thr Phe Gln Gln Tyr
    130                 135                 140

Trp Ser Val Arg Thr Ser Lys Arg Thr Ser Gly Thr Ile Ser Val Thr
145                 150                 155                 160

Glu His Phe Lys Gln Trp Glu Arg Met Gly Met Arg Met Gly Lys Met
                165                 170                 175

Tyr Glu Val Ala Leu Thr Val Glu Gly Tyr Gln Ser Ser Gly Tyr Ala
            180                 185                 190

Asn Val Tyr Lys Asn Glu Ile Arg Ile Gly Ala Asn
        195                 200


<210>  4
<211>  44
<212>  DNA
<213>  Artificial sequence

<220>
<223>  Synthetic oligonucleotide

<400>  4
ttaagaagga gatatacata tggggcgaat aatttacgac aatg                        44


<210>  5
<211>  44
<212>  DNA
<213>  Artificial sequence

<220>
<223>  Synthetic oligonucleotide

<400>  5
gtggtggtgg tggtgctcga gagttcctga ttttgagaat acaa                        44


<210>  6
<211>  52
<212>  DNA
<213>  Artificial sequence

<220>
<223>  Synthetic oligonucleotide

<400>  6
atctcagtgg tggtggtggt ggtgctcgag tgaacttctg ctaaatatga ag               52


<210>  7
<211>  51
<212>  DNA
<213>  Artificial sequence

<220>
```

<223> Synthetic oligonucleotide

<400> 7
atctcagtgg tggtggtggt ggtgctcgag atttgcacct attctgattt c 51

<210> 8
<211> 1863
<212> DNA
<213> Clostridium stercorarium

<400> 8
gggcgaataa tttacgacaa tgagacaggc acacatggag gctacgacta tgagctctgg 60

aaagactacg gaaatacgat tatggaactt aacgacggtg gtacttttag ttgtcaatgg 120

agtaatatcg gtaatgcact atttagaaaa gggagaaaat ttaattccga caaaacctat 180

caagaattag gagatatagt agttgaatat ggctgtgatt acaatccaaa cggaaattcc 240

tatttgtgtg tttacggttg gacaagaaat ccactggttg aatattacat tgtagaaagc 300

tggggcagct ggcgtccacc tggagcaaca cccaaaggaa ccatcacagt ggatggcggt 360

acttatgaaa tatatgaaac tacccgggta aatcagcctt ccatcgatgg aactgcgaca 420

ttccaacaat attggagtgt tcgtacatcc aagagaacaa gcggaacaat atctgtcact 480

gaacatttta aacagtggga aagaatgggc atgcgaatgg gtaagatgta tgaagttgct 540

cttaccgttg aaggttatca gagcagtggg tacgctaatg tatacaagaa tgaaatcaga 600

ataggtgcaa atccaactcc tgccccatct caaagcccaa ttagaagaga tgcattttca 660

ataatcgaag cggaagaata taacagcaca aattcctcca ctttacaagt gattggaacg 720

ccaaataatg gcagaggaat tggttatatt gaaaatggta ataccgtaac ttacagcaat 780

atagattttg gtagtggtgc aacagggttc tctgcaactg ttgcaacgga ggttaatacc 840

tcaattcaaa tccgttctga cagtcctacc ggaactctac ttggtacctt atatgtaagt 900

tctaccggca gctggaatac atatcaaacc gtatctacaa acatcagcaa aattaccggc 960

gttcatgata ttgtattggt attctcaggt ccagtcaatg tggacaactt catatttagc 1020

agaagttcac cagtgcctgc acctggtgat aacacaagag acgcatattc tatcattcag 1080

gccgaggatt atgacagcag ttatggcccc aaccttcaaa tctttagctt accaggcggt 1140

ggcagcgcca ttggctatat tgaaaatggt tattccacta cctataataa cgttaatttc 1200

gccaacggct taagttctat aacagcaaga gttgccactc agatctcaac ttccattcag 1260

gtgagagcag gaggagcaac cggtacttta cttggtacaa tatatgttcc ttcgacaaat 1320

agttgggatt cttatcagaa tgtaactgcc aaccttagca atattacagg tgtgcatgat 1380

attacccttg tcttttcagg accagtgaat gtggactact tcgtatttac accagcaaat 1440

gtaaattcag ggcctacctc ccctgtcgga ggtacaagaa gtgcattttc caatattcaa 1500

gccgaagatt atgacagcag ttatggtccc aaccttcaaa tctttagctt accaggtggt 1560

ggcagcgcca ttggctatat tgaaaatggt tattccacta cctataaaaa tattgatttt 1620

ggtgacggcg caacgtccgt aacagcaaga gtagctaccc agaatgctac taccattcag 1680

gtaagattgg gaagtccatc gggtacatta cttggaacaa tttacgtggg gtccacagga 1740

agctttgata cttataggga tgtatccgct accattagta atactgcggg tgtaaaagat 1800

attgttcttg tattctcagg tcctgttaat gttgactggt ttgtattctc aaaatcagga 1860

act 1863

<210> 9
<211> 1029
<212> DNA
<213> Artificial sequence

<220>
<223> Synthetic or recombinant polynucleotide

<400> 9
gggcgaataa tttacgacaa tgagacaggc acacatggag gctacgacta tgagctctgg 60

aaagactacg gaaatacgat tatggaactt aacgacggtg gtacttttag ttgtcaatgg 120

agtaatatcg gtaatgcact atttagaaaa gggagaaaat ttaattccga caaaacctat 180

caagaattag gagatatagt agttgaatat ggctgtgatt acaatccaaa cggaaattcc 240

tatttgtgtg tttacggttg gacaagaaat ccactggttg aatattacat tgtagaaagc 300

tggggcagct ggcgtccacc tggagcaaca cccaaaggaa ccatcacagt ggatggcggt 360

acttatgaaa tatatgaaac tacccgggta aatcagcctt ccatcgatgg aactgcgaca 420

ttccaacaat attggagtgt tcgtacatcc aagagaacaa gcggaacaat atctgtcact 480

gaacatttta aacagtggga aagaatgggc atgcgaatgg gtaagatgta tgaagttgct 540

cttaccgttg aaggttatca gagcagtggg tacgctaatg tatacaagaa tgaaatcaga 600

ataggtgcaa atccaactcc tgccccatct caaagcccaa ttagaagaga tgcattttca 660

ataatcgaag cggaagaata taacagcaca aattcctcca ctttacaagt gattggaacg 720

ccaaataatg gcagaggaat tggttatatt gaaaatggta ataccgtaac ttacagcaat 780

atagattttg gtagtggtgc aacagggttc tctgcaactg ttgcaacgga ggttaatacc 840

tcaattcaaa tccgttctga cagtcctacc ggaactctac ttggtacctt atatgtaagt 900

tctaccggca gctggaatac atatcaaacc gtatctacaa acatcagcaa aattaccggc 960

gttcatgata ttgtattggt attctcaggt ccagtcaatg tggacaactt catatttagc 1020

agaagttca 1029

<210> 10
<211> 612
<212> DNA

<213> Artificial sequence

<220>
<223> Synthetic or recombinant polynucleotide

<400> 10
gggcgaataa tttacgacaa tgagacaggc acacatggag gctacgacta tgagctctgg 60

aaagactacg gaaatacgat tatggaactt aacgacggtg gtacttttag ttgtcaatgg 120

agtaatatcg gtaatgcact atttagaaaa gggagaaaat ttaattccga caaaacctat 180

caagaattag gagatatagt agttgaatat ggctgtgatt acaatccaaa cggaaattcc 240

tatttgtgtg tttacggttg gacaagaaat ccactggttg aatattacat tgtagaaagc 300

tggggcagct ggcgtccacc tggagcaaca cccaaaggaa ccatcacagt ggatggcggt 360

acttatgaaa tatatgaaac tacccgggta aatcagcctt ccatcgatgg aactgcgaca 420

ttccaacaat attggagtgt tcgtacatcc aagagaacaa gcggaacaat atctgtcact 480

gaacatttta aacagtggga aagaatgggc atgcgaatgg gtaagatgta tgaagttgct 540

cttaccgttg aaggttatca gagcagtggg tacgctaatg tatacaagaa tgaaatcaga 600

ataggtgcaa at 612

<210> 11
<211> 534
<212> PRT
<213> Artificial sequence

<220>
<223> Recombinant polypeptide

<400> 11

Ala Lys Ile Thr Glu Asn Tyr Gln Phe Asp Ser Arg Ile Arg Leu Asn
1 5 10 15

Ser Ile Gly Phe Ile Pro Asn His Ser Lys Lys Ala Thr Ile Ala Ala
20 25 30

Asn Cys Ser Thr Phe Tyr Val Val Lys Glu Asp Gly Thr Ile Val Tyr
35 40 45

Thr Gly Thr Ala Thr Ser Met Phe Asp Asn Asp Thr Lys Glu Thr Val
50 55 60

Tyr Ile Ala Asp Phe Ser Ser Val Asn Glu Glu Gly Thr Tyr Tyr Leu
65 70 75 80

Ala Val Pro Gly Val Gly Lys Ser Val Asn Phe Lys Ile Ala Met Asn
85 90 95

```
Val Tyr Glu Asp Ala Phe Lys Thr Ala Met Leu Gly Met Tyr Leu Leu
            100                 105                 110

Arg Cys Gly Thr Ser Val Ser Ala Thr Tyr Asn Gly Ile His Tyr Ser
        115                 120                 125

His Gly Pro Cys His Thr Asn Asp Ala Tyr Leu Asp Tyr Ile Asn Gly
    130                 135                 140

Gln His Thr Lys Lys Asp Ser Thr Lys Gly Trp His Asp Ala Gly Asp
145                 150                 155                 160

Tyr Asn Lys Tyr Val Val Asn Ala Gly Ile Thr Val Gly Ser Met Phe
                165                 170                 175

Leu Ala Trp Glu His Phe Lys Asp Gln Leu Glu Pro Val Ala Leu Glu
            180                 185                 190

Ile Pro Glu Lys Asn Asn Ser Ile Pro Asp Phe Leu Asp Glu Leu Lys
        195                 200                 205

Tyr Glu Ile Asp Trp Ile Leu Thr Met Gln Tyr Pro Asp Gly Ser Gly
    210                 215                 220

Arg Val Ala His Lys Val Ser Thr Arg Asn Phe Gly Gly Phe Ile Met
225                 230                 235                 240

Pro Glu Asn Glu His Asp Glu Arg Phe Phe Val Pro Trp Ser Ser Ala
                245                 250                 255

Ala Thr Ala Asp Phe Val Ala Met Thr Ala Met Ala Ala Arg Ile Phe
            260                 265                 270

Arg Pro Tyr Asp Pro Gln Tyr Ala Glu Lys Cys Ile Asn Ala Ala Lys
        275                 280                 285

Val Ser Tyr Glu Phe Leu Lys Asn Asn Pro Ala Asn Val Phe Ala Asn
    290                 295                 300

Gln Ser Gly Phe Ser Thr Gly Glu Tyr Ala Thr Val Ser Asp Ala Asp
305                 310                 315                 320

Asp Arg Leu Trp Ala Ala Ala Glu Met Trp Glu Thr Leu Gly Asp Glu
                325                 330                 335

Glu Tyr Leu Arg Asp Phe Glu Asn Arg Ala Ala Gln Phe Ser Lys Lys
            340                 345                 350
```

```
Ile Glu Ala Asp Phe Asp Trp Asp Asn Val Ala Asn Leu Gly Met Phe
        355                 360                 365

Thr Tyr Leu Leu Ser Glu Arg Pro Gly Lys Asn Pro Ala Leu Val Gln
    370                 375                 380

Ser Ile Lys Asp Ser Leu Leu Ser Thr Ala Asp Ser Ile Val Arg Thr
385                 390                 395                 400

Ser Gln Asn His Gly Tyr Gly Arg Thr Leu Gly Thr Thr Tyr Tyr Trp
                405                 410                 415

Gly Cys Asn Gly Thr Val Val Arg Gln Thr Met Ile Leu Gln Val Ala
            420                 425                 430

Asn Lys Ile Ser Pro Asn Asn Asp Tyr Val Asn Ala Ala Leu Asp Ala
        435                 440                 445

Ile Ser His Val Phe Gly Arg Asn Tyr Tyr Asn Arg Ser Tyr Val Thr
    450                 455                 460

Gly Leu Gly Ile Asn Pro Pro Met Asn Pro His Asp Arg Arg Ser Gly
465                 470                 475                 480

Ala Asp Gly Ile Trp Glu Pro Trp Pro Gly Tyr Leu Val Gly Gly Gly
                485                 490                 495

Trp Pro Gly Pro Lys Asp Trp Val Asp Ile Gln Asp Ser Tyr Gln Thr
            500                 505                 510

Asn Glu Ile Ala Ile Asn Trp Asn Ala Ala Leu Ile Tyr Ala Leu Ala
        515                 520                 525

Gly Phe Val Asn Tyr Asn
    530

<210>  12
<211>  1602
<212>  DNA
<213>  Artificial sequence

<220>
<223>  Synthetic polynucleotide

<400>  12
gcaaaaataa cggagaatta tcaatttgat tcacgaatcc gtttaaactc aataggtttt        60

ataccgaacc acagcaaaaa ggcgactata gctgcaaatt gttcaacctt ttatgttgtt       120

aaagaagacg gaacaatagt gtataccgga acggcaactt caatgtttga caatgataca       180
```

```
aaagaaactg tttatattgc tgatttttca tctgttaatg aagaaggaac gtactatctt      240
gccgtgccgg gagtaggaaa aagcgtaaac tttaaaattg caatgaatgt atatgaggat      300
gcttttaaaa cagcaatgct gggaatgtat ttgctgcgct gcggcaccag tgtgtcggcc      360
acatacaacg gaatacacta ttcccatgga ccgtgccata ctaatgatgc atatcttgat      420
tatataaacg gacagcatac taaaaaagac agtacaaaag gctggcatga tgcgggcgac      480
tacaacaaat atgtggtaaa cgccggcata accgttggtt caatgttcct ggcgtgggag      540
cattttaaag accagttgga gcctgtggca ttggagattc ccgaaaagaa caattcaata      600
ccggattttc ttgatgaatt aaaatatgag atagactgga ttcttaccat gcaataccct      660
gacgggagcg gaagggtggc tcataaagtt tcgacaagga actttggcgg ctttatcatg      720
cctgagaacg aacacgacga aagatttttc gtgccctgga gcagtgccgc aacggcagac      780
tttgttgcca tgacggccat ggctgcaaga atattcaggc cttatgatcc tcaatatgct      840
gaaaaatgta taaatgcggc aaaagtaagc tatgagtttt tgaagaacaa tcctgcgaat      900
gtttttgcaa accagagtgg attctcaaca ggagaatatg ccactgtcag tgatgcagat      960
gacagattgt gggcggcggc tgaaatgtgg gagaccctgg gagatgaaga ataccttaga     1020
gattttgaaa acagggcggc gcaattctcg aaaaaaatag aagccgattt tgactgggat     1080
aatgttgcaa acttaggtat gtttacatat cttttgtcag aaagaccggg caagaatcct     1140
gctttggtgc agtcaataaa ggatagtctc ctttccactg cggattcaat tgtgaggacc     1200
agccaaaacc atggctatgg cagaaccctt ggtacaacat attactgggg atgcaacggc     1260
acggttgtaa gacagactat gatacttcag gttgcgaaca agatttcacc caacaatgat     1320
tatgtaaatg ctgctctcga tgcgatttca catgtatttg gaagaaacta ttacaacagg     1380
tcttatgtaa caggccttgg tataaatcct cctatgaatc ctcatgacag acgttcaggg     1440
gctgacggaa tatgggagcc gtggcccggt taccttgtag gaggaggatg gcccggaccg     1500
aaggattggg tggatattca ggacagttat cagaccaatg aaattgctat aaactggaat     1560
gcggcattga tttatgccct tgccggattt gtcaactata at                        1602
```